(19) Europäisches Patentamt
European Patent Office
Office européen des brevets

(11) **EP 4 628 546 A1**

(12) **EUROPEAN PATENT APPLICATION**

(43) Date of publication:
**08.10.2025 Bulletin 2025/41**

(21) Application number: **24305532.4**

(22) Date of filing: **04.04.2024**

(51) International Patent Classification (IPC):
***C09B 23/01*** *(2006.01)* ***C09B 23/08*** *(2006.01)*
***A61P 35/00*** *(2006.01)*

(52) Cooperative Patent Classification (CPC):
**C09B 23/086; A61P 35/00; C09B 23/0066**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Centre National de la Recherche Scientifique 75016 Paris (FR)**
- **Université de Lorraine 54000 Nancy (FR)**

(72) Inventors:
- **Lamande-Langle, Sandrine Tomblaine (FR)**
- **Pellegrini-Moise, Nadia Vandoeuvre-lès-Nancy (FR)**
- **Jouad, Kamal Memphis (US)**
- **Bernhard, Yann Saulxures-lès-Nancy (FR)**
- **Selmeczi, Katalin Fléville-devant-Nancy (FR)**

(74) Representative: **Bandpay & Greuter 11 rue Christophe Colomb 75008 Paris (FR)**

(54) **CYANINE DIMERS FOR PHOTOTHERMAL TREATMENT OF CANCER GUIDED BY FLUORESCENCE/PHOTOACOUSTIC IMAGING**

(57) The present invention relates to compounds of formula (I) and its use for photothermal therapy preferably for the treatment of cancer, for examples for the treatment of glioblastoma, prostate cancer, breast cancer and melanoma

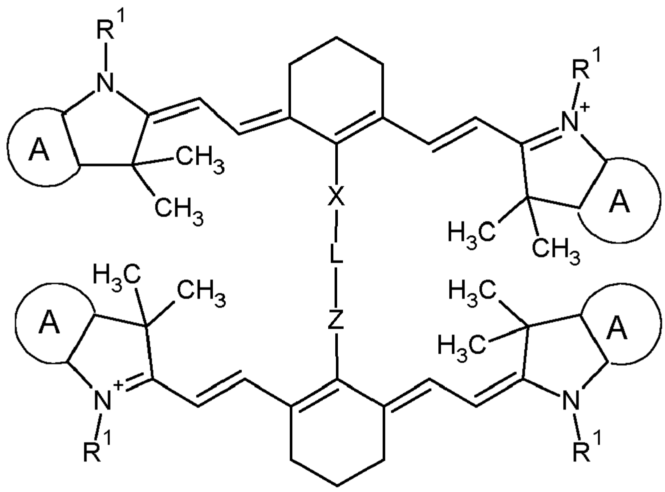

, 2 D⁻ (I).

EP 4 628 546 A1

## Description

### Technical field

The present invention relates to cyanine dimers for diagnosis of cancer and photothermal treatment of cancer guided by fluorescence and/or photoacoustic imaging.

### Background art

Photothermal treatment is a therapeutical approach based on light for the treatment of various cancers. This approach is based on a local increase of the temperature by irradiation of a photothermal agent (PTA) at a specific wavelength. The photoacoustic imaging is a non-invasive technique based on the detection of photo-induced ultrasound signals. This technique combines the elevated contrast of near-infrared fluorescence imaging with the deep penetrability of ultrasound imaging. Fluorescence imaging uses fluorophore absorbing and emitting in near-infrared. A combined use of these therapy and imaging methods enable a photothermal therapy guided by photoacoustic and fluorescence imaging in near-infrared region. Photoacoustic imaging and photothermal treatment require photoactive compounds having low fluorescence quantum yield whereas the fluorescence imaging requires high fluorescence quantum yield and brightness.
Cyanine derivatives are used in photothermal therapy in preclinical applications. However, as other organic fluorophore, their photothermal activity is limited, they are not sufficiently stable in aqueous solution and have poor stability under irradiation. Moreover, theses compounds do not enable a specific targeting of tumors.
There is thus a need to provide compounds having a compromise of properties in order to meet the requirements of photoacoustic imaging or/and fluorescence imaging and photothermal treatment.
Moreover, there is a need to provide compounds that can be easily modified to enhance their biodisponibility, for example by increasing their hydrosolubility and/or to graft some targeting molecules to specifically target some cancer cells, tumor, etc.

### Summary of the invention

The invention relates to compound of formula (I)

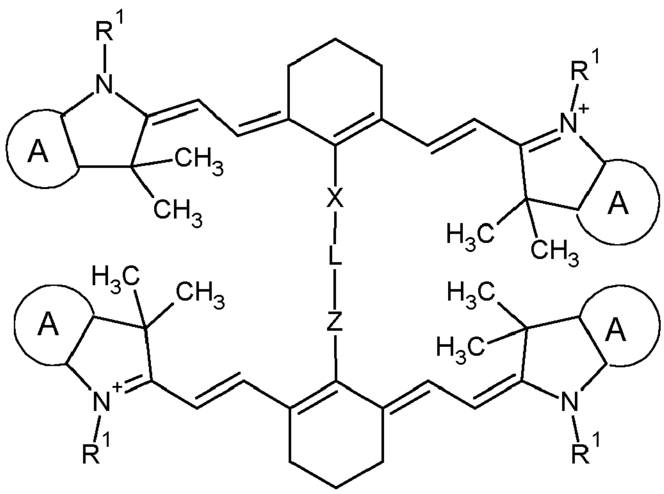

, 2 $D^-$ (I)

wherein

- A represents a phenyl or naphtyl group, optionally substituted;
- D, each identical or different, represents an anion;
- $R^1$ represents a propyl group or a butyl-$SO_3M$ group with M is H, an ammonium group or a cation;
- X is NH, N(Alk), S or O, with Alk is an alkyl group, linear or branched, comprising from 1 to 10 carbon atoms, preferably from 1 to 5 carbon atoms, preferably $CH_3$,
- Z is NH, N(Alk), S or O, with Alk is an alkyl group, linear or branched, comprising from 1 to 10 carbon atoms, preferably from 1 to 5 carbon atoms, preferably $CH_3$,
- L is $(CH_2)_n$-Ph-$(CH_2)_{n'}$ wherein the phenyl is substituted by a COR' group; $(CH_2)_m$-(triazole)-$(CH_2)_P$-C(O)N($CH_3$)$(CH_2)_{m'}$; alkyl linear or branched comprising from 1 to 8 carbon atoms; alkyl, linear or branched, comprising from 1 to 8

carbon atoms and substituted by one COR' group; $(CH_2)_m$-N($CH_3$)-C(O)-$(CH_2)_q$-(triazole)-$(CH_2)_p$-C(O)-N($CH_3$)-$(CH_2)_{m'}$,

- p is an integer between 1 and 5, preferably between 2 and 4,
- q is an integer between 1 and 5, preferably between 2 and 4,
- m is an integer between 1 to 8,
- m' is an integer between 1 to 8,
- n is an integer between 0 and 2,
- n' is an integer between 0 and 2;
- R' is OH or R;
- R is is NH-$(CH_2)_r$-triazole-$(CH_2)_2$-O-($CH_2$-$CH_2$-O)$_x$-$CH_2CH_2$C(O)NH-W; NH$(CH_2)_r$-triazole-$(CH_2)_s$-(polyhydroxylated 5 or 6-cycloalkyl comprising an oxygen atom)-OW; NH$(CH_2)_r$-triazole-$(CH_2)_s$-(polyhydroxylated 5 or 6-cycloalkyl comprising an oxygen atom)-OH; NH-$(CH_2)_r$-triazole-$(CH_2)_2$-O-($CH_2$-$CH_2$-O)$_x$$(CH_2)_2$-COOH; NH-$(CH_2)_r$-triazole-$(CH_2)_2$-O-($CH_2$-$CH_2$-O)$_x$$(CH_2)_2$-C(O)-W; NH-alkyl-C=CH wherein the alkyl is linear or branched and comprises from 2 to 5 carbon atoms; NH-alkyl-$N_3$ wherein the alkyl is linear or branched and comprises from 2 to 5 carbon atoms; NH-alkyl-NH-Q wherein the alkyl is linear or branched and comprises from 2 to 5 carbon atoms; NH-$(CH_2)_2$-O-($CH_2$-$CH_2$-O)$_x$-$CH_2CH_2$-COOH; NH-$(CH_2)_2$-O-($CH_2$-$CH_2$-O)$_x$-$CH_2CH_2$-NH-Q; NH-$(CH_2)_2$-O-($CH_2$-$CH_2$-O)$_x$-$CH_2CH_2$-CO-NH-W; NH-$(CH_2)_2$-O-($CH_2$-$CH_2$-O)$_X$-$CH_2CH_2$-NH-W; W; PEG group; solubilizing group;

Q is H or a protective group;
r is an integer between 2 to 5,
s is an integer between 2 to 5,
x is an integer between 2 to 8,
W is a bioactive compound or a group comprising a bioactive compound;

with the proviso that when X=Z=O, NH or S, L is not an alkyl and its pharmaceutically acceptable salts.

Preferably, in the compound of formula (I) X=Z L is not $CH_2$-Ph-$CH_2$.

Preferably, in the compound of formula (I) X is NH, S or O, Z is NH, S or O and X and Z are different.

Preferably A is a phenyl group.

Preferably, the compounds of formula (I) are chosen among the following compounds:

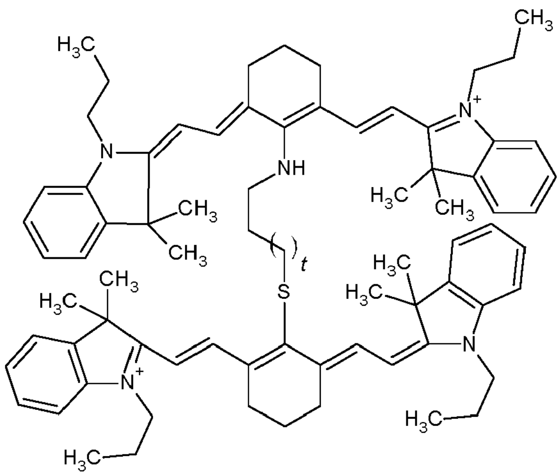

, 2D$^-$,

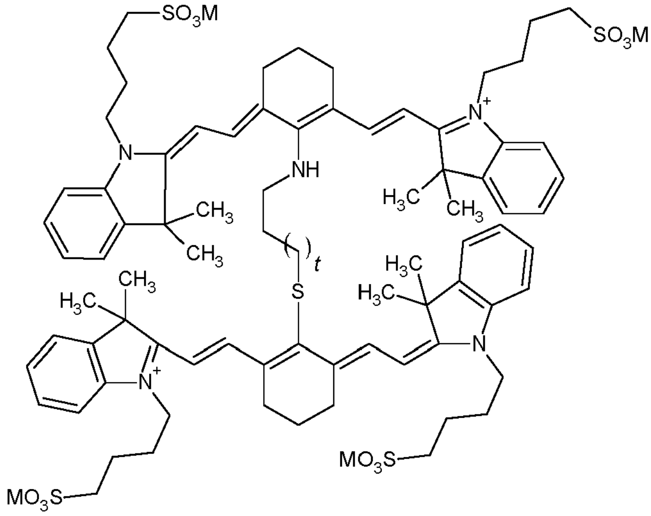

, $2D^-$

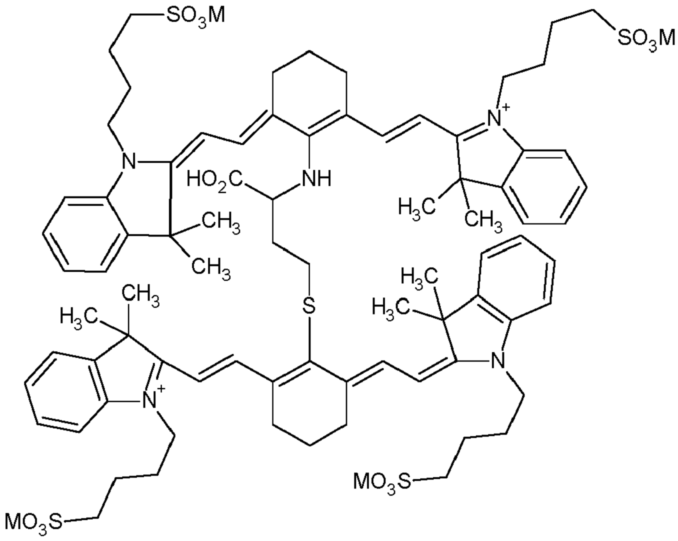

, $2D^-$

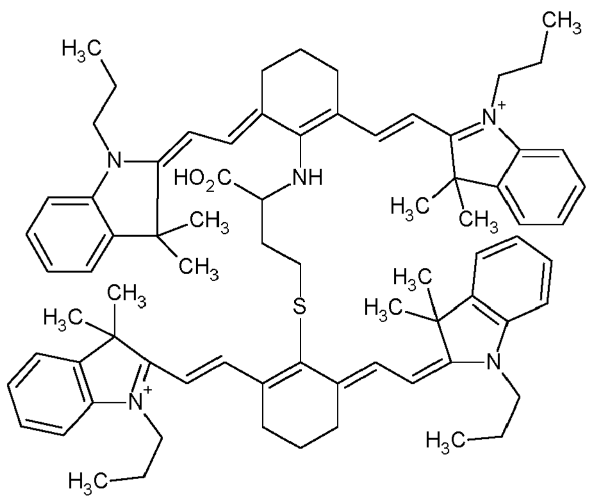

, $2D^-$

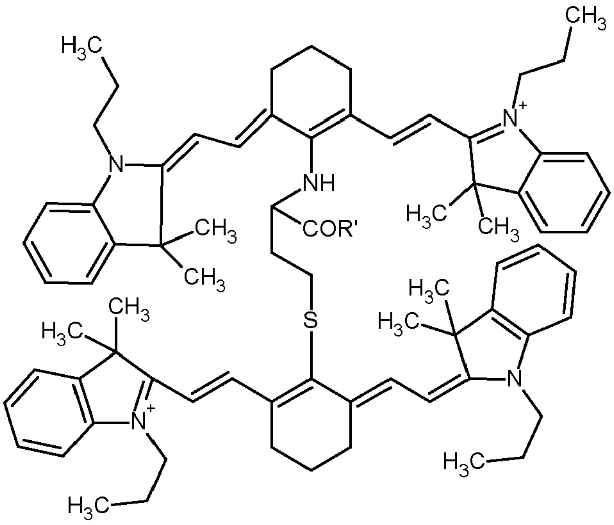

, $2D^-$

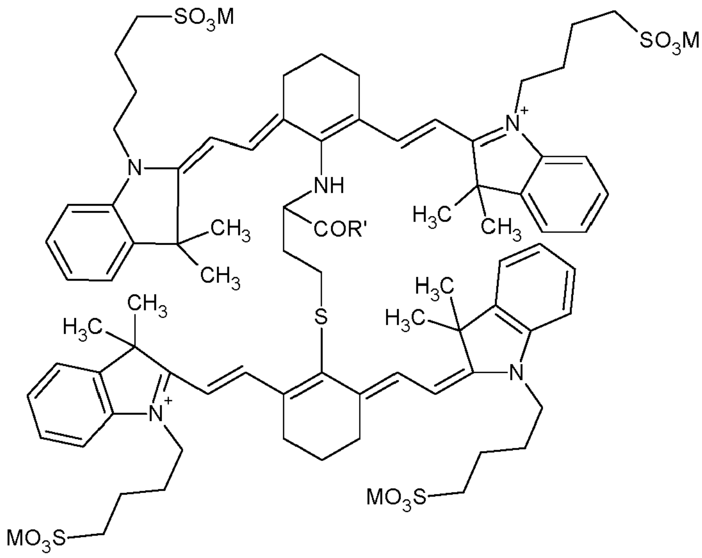

, $2D^-$

R' is NH-$(CH_2)_{1-3}$-$(CH_2)$-C≡CH, NH-$(CH_2)_{1-3}$-$(CH_2)$-NH-protection, NH-$(CH_2)_{1-3}$-$CH_2$-(triazole)-$CH_2$-$CH_2$-O($CH_2$-$CH_2$-O)$_{3-7}$-$CH_2$-$CH_2$-COOH, NH-$(CH_2)_{1-3}$-$CH_2$-(triazole)-$CH_2$-$CH_2$-$CH_2$-(polyhydroxylated 5 or 6-cycloalkyl comprising an oxygen atom)-OH, NH-$(CH_2)_{1-3}$-$CH_2$-(triazole)-$CH_2$-$CH_2$-$CH_2$-(polyhydroxylated 5 or 6-cycloalkyl comprising an oxygen atom)-OW, NH-$(CH_2)_{1-3}$-$CH_2$-(triazole)-$CH_2$-$CH_2$-O($CH_2$-$CH_2$-O)$_{3-7}$-$CH_2$-$CH_2$-CONHW; NH-$(CH_2)_2$-O-($CH_2$-$CH_2$-O)$_x$-$CH_2CH_2$-COOH; NH-$(CH_2)_2$-O-($CH_2$-$CH_2$-O)$_x$-$CH_2CH_2$-NH-Q; NH-$(CH_2)_2$-O-($CH_2$-$CH_2$-O)$_x$-$CH_2CH_2$-CO-NH-W; NH-$(CH_2)_2$-O-($CH_2$-$CH_2$-0)$_x$-$CH_2CH_2$-NH-W; x, W and Q being as defined above ;

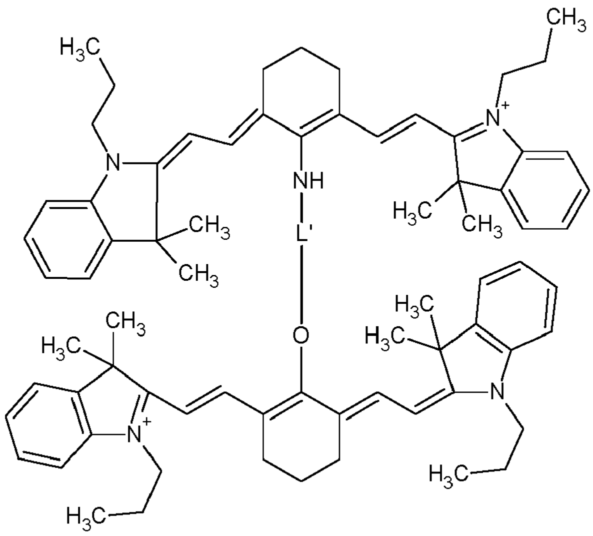

, $2D^-$

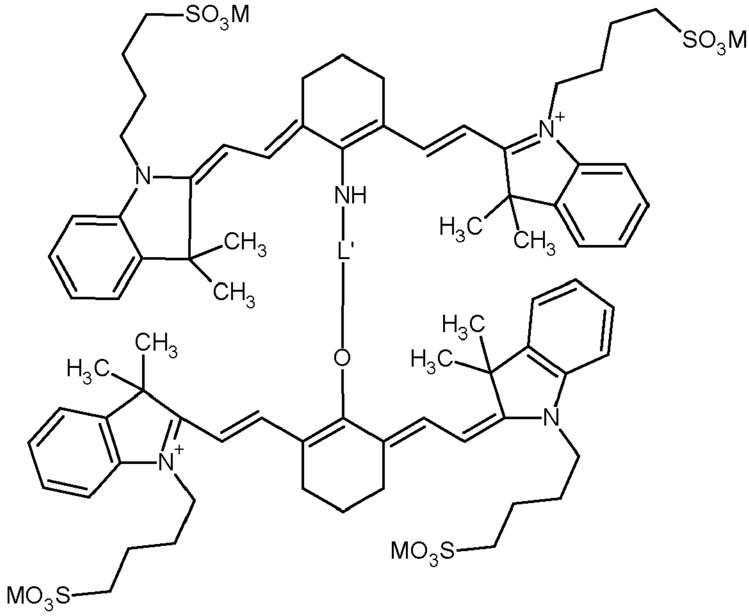

, $2D^-$

L' is $(CH_2)_m$-(triazole)-$(CH_2)_P$-$C(O)N(CH_3)(CH_2)_{m'}$; p, m and m' being as defined above

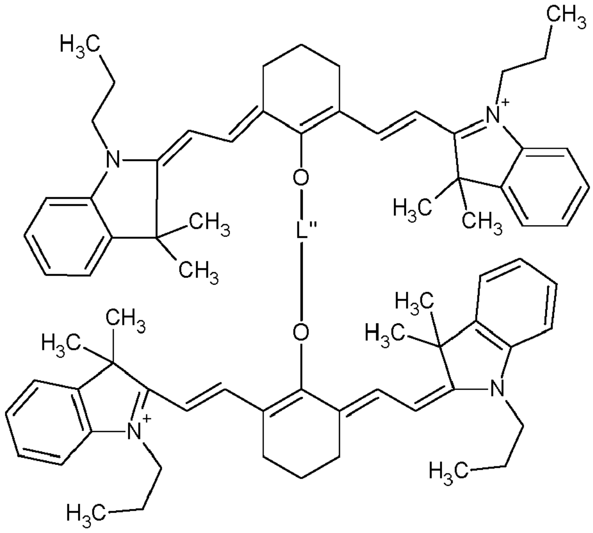

, $2D^-$

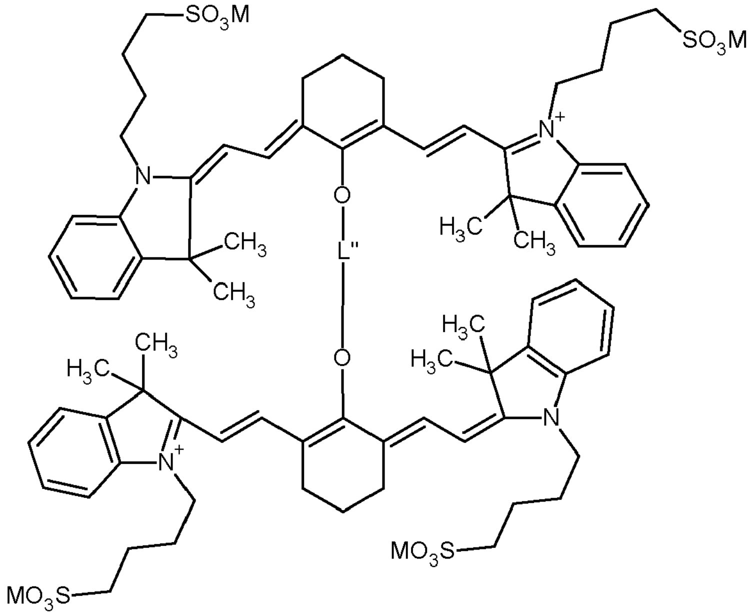

, $2D^-$

L'' is $(CH_2)_m$-$N(CH_3)$-C(O)-$(CH_2)_q$-(triazole)-$(CH_2)_p$-C(O)-$N(CH_3)$-$(CH_2)_{m'}$, p, q, m and m' being as defined above

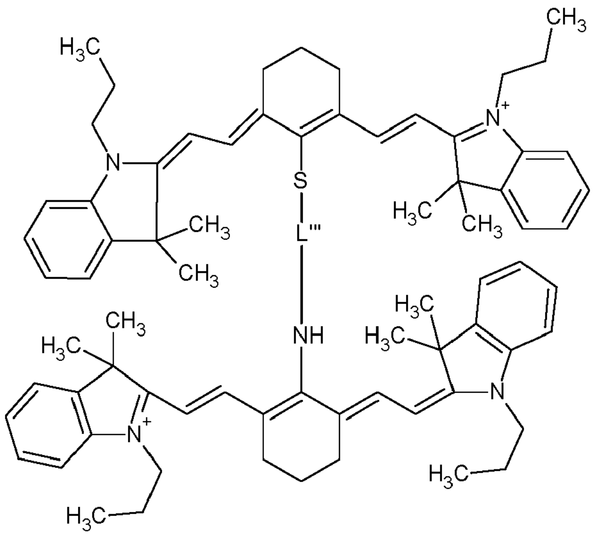

, $2D^-$

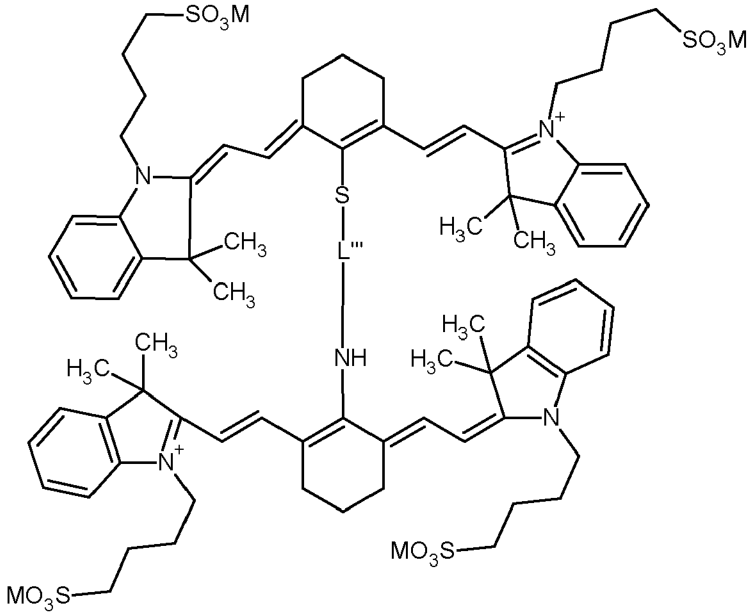

, $2D^-$

L''' is $(CH_2)_n$-Ph-$(CH_2)_{n'}$ wherein the phenyl is substituted by a COR' group; R' is OH or R, preferably OH; R, n and n' being as defined above
wherein t is an integer from 0 to 6, D each identical or different represents an anion, preferably I; M is H, an ammonium group or a cation, preferably Na or K.

The invention also relates to a composition comprising at least one compound of formula (I) and optionally at least one pharmaceutically acceptable excipient.

The present invention also relates to the compound or composition defined above for use for the diagnosis of tumor by fluorescent imaging and/or photoacoustic imaging, preferably for use for the treatment of cancer or microbial infection, preferably for the treatment of Glioblastoma, prostate cancer, breast cancer and melanoma.

The present invention also relates to the compound or composition defined above for use for phothermal therapy, preferably for the treatment of cancer, preferably for use for photothermal therapy guides by fluorescent or/and photoacoustic imaging.

## Detailed description

The present invention relates to compounds of formula (I)

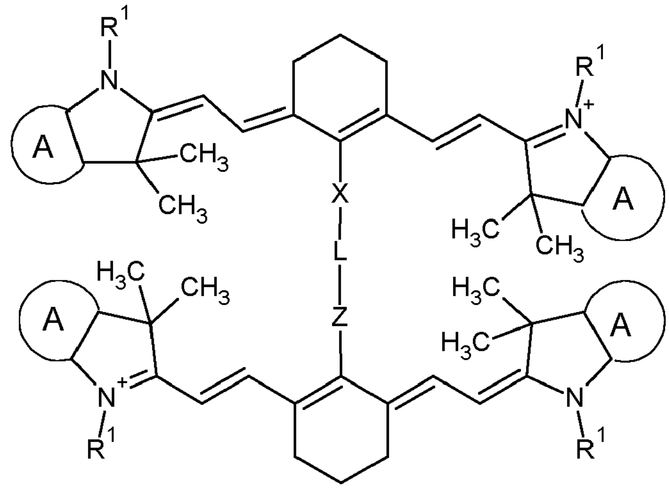

, $2 D^-$ (I)

wherein

A represents a phenyl or naphtyl group, optionally substituted;
D, each identical or different, represents an anion;
$R^1$ represents a propyl group or a butyl-$SO_3M$ group with M is H, an ammonium group or a cation;
X is NH, N(Alk), S or O, with Alk is an alkyl group, linear or branched, comprising from 1 to 10 carbon atoms, preferably from 1 to 5 carbon atoms, preferably $CH_3$,
Z is NH, N(Alk), S or O, with Alk is an alkyl group, linear or branched, comprising from 1 to 10 carbon atoms, preferably from 1 to 5 carbon atoms, preferably $CH_3$,
L is $(CH_2)_n$-Ph-$(CH_2)_{n'}$ wherein the phenyl is substituted by a COR' group;
$(CH_2)_m$-(triazole)-$(CH_2)_P$-C(O)N($CH_3$)$(CH_2)_{m'}$; alkyl linear or branched comprising from 1 to 8 carbon atoms; alkyl, linear or branched, comprising from 1 to 8 carbon atoms and substituted by one COR' group; $(CH_2)_m$-N($CH_3$)-C(O)-$(CH_2)_q$-(triazole)-$(CH_2)_p$-C(O)-N($CH_3$)-$(CH_2)_{m'}$,
p is an integer between 1 and 5, preferably between 2 and 4,
q is an integer between 1 and 5, preferably between 2 and 4,
m is an integer between 1 to 8,
m' is an integer between 1 to 8,
n is an integer between 0 and 2,
n' is an integer between 0 and 2;
R' is OH or R;
R is NH-$(CH_2)_r$-triazole-$(CH_2)_2$-O-($CH_2$-$CH_2$-O$)_x$-$CH_2CH_2$C(O)NH-W; NH$(CH_2)_r$-triazole-$(CH_2)_s$-(polyhydroxylated 5 or 6-cycloalkyl comprising an oxygen atom)-OW; NH$(CH_2)_r$-triazole-$(CH_2)_s$-(polyhydroxylated 5 or 6-cycloalkyl comprising an oxygen atom)-OH; NH-$(CH_2)_r$-triazole-$(CH_2)_2$-O-($CH_2$-$CH_2$-O$)_x$$(CH_2)_2$-COOH; NH-$(CH_2)_r$-triazole-$(CH_2)_2$-O-($CH_2$-$CH_2$-O$)_x$$(CH_2)_2$-C(O)-W; NH-alkyl-C=CH wherein the alkyl is linear or branched and comprises from 2 to 5 carbon atoms; NH-alkyl-$N_3$ wherein the alkyl is linear or branched and comprises from 2 to 5 carbon atoms; NH-alkyl-NH-Q wherein the alkyl is linear or branched and comprises from 2 to 5 carbon atoms; NH-$(CH_2)_2$-O-($CH_2$-$CH_2$-O$)_x$-$CH_2CH_2$-COOH; NH-$(CH_2)_2$-O-($CH_2$-$CH_2$-O$)_x$-$CH_2CH_2$-NH-Q; NH-(CHz)z-O-($CH_2$-$CH_2$-O$)_x$-$CH_2CH_2$-CO-NH-W; NH-$(CH_2)_2$-O-($CH_2$-$CH_2$-O$)_X$-$CH_2CH_2$-NH-W;
W; PEG group; a solubilizing group;
Q is H or a protective group;
r is an integer between 2 to 5,
s is an integer between 2 to 5,
x is an integer between 2 to 8,
W is a bioactive compound or a group comprising a bioactive compound; with the proviso that when X=Z=O, NH or S, L is not an alkyl

and its pharmaceutically acceptable salts.

In the present invention, a bioactive compound is a compound having a biological activity, i.e. a compound that targets cellular receptors, and more preferably cellular receptor of cancer cells and/or tumors. Examples of bioactive compounds are peptides, proteins, vitamins or vitamin derivatives, for example biotine, folate, polysaccharide, sugar, FAP inhibitors (FAP = Fibroblast Activation Protein), DUPA inhibitors (DUPA = 2-[3-(1,3-dicarboxypropyl)ureido]pentanedioic

acid). In the present invention, the peptide can be any peptide and specifically peptide targeting cellular receptor and more preferably tumoral receptor, an example of such peptide can be peptide targeting $\alpha v\beta 3$ integrin.

In the present invention, a group comprising a bioactive compound is a group comprising a linker and a bioactive compound such as defined above, wherein the linker enables to link the bioactive compound to the compound of formula (I), such link can be chosen among carbohydrate, PEG group or an alkyl group comprising an amide group or a triazole group.

In the present invention, a protective group is a group that renders the amine non-reactive, such protective group are known by the skilled person and can for example be chosen among tert-butyloxycarbonyle (Boc), benzyloxycarbonyl (Cbz), fluorenylmethoxycarbonyle (Fmoc).

The term "solubilizing group" refers to a group that improves the hydrosolubility of the compound. The increase of the hydrosolubility enables to improve the biodisponibility. As an example of solubilizing group it can be mentioned polyethylene glycol group (PEG), carbohydrate.

In one embodiment, in the compound of formula (I), X and Z are different. Preferably, in the compound of formula (I) of the invention, A is a phenyl group.

In one embodiment, in the compound of formula (I) X and Z are different and A is a phenyl group.

Preferably, in the compound of formula (I) $R^1$ represents a propyl group or a butyl-$SO_3M$ group with M is H, an ammonium group or a cation chosen among sodium and potassium.

Preferably, in the compounds of formula (I) D is chosen among iodide, chloride, bromide, triflate, trifluoroacetate, perchlorate.

Preferably, in the compound of formula (I) X is NH, N(Alk) with Alk is an alkyl group, linear or branched, comprising from 1 to 10 carbon atoms, preferably from 1 to 5 carbon atoms, preferably $CH_3$ and Z is NH, N(Alk), S or O, with Alk is an alkyl group, linear or branched, comprising from 1 to 10 carbon atoms, preferably from 1 to 5 carbon atoms, preferably $CH_3$.

Preferably, in the compound of formula (I), L is $(CH_2)_2$-Ph-$(CH_2)_2$ wherein the phenyl is substituted by a COR' group; $(CH_2)_3$-(triazole)-$(CH_2)_3$-C(O)N($CH_3$)$(CH_2)_3$; alkyl linear or branched comprising from 1 to 8 carbon atoms; alkyl, linear or branched, comprising from 1 to 8 carbon atoms and substituted by one COR' group; $(CH_2)_3$-N($CH_3$)-C(O)-$(CH_2)_5$-(triazole)-$(CH_2)_2$-C(O)-N($CH_3$)-$(CH_2)_3$,

R' is OH or R;

R is NH-$(CH_2)_2$-triazole-$(CH_2)_2$-O-($CH_2$-$CH_2$-O$)_3$-$CH_2CH_2$C(O)NH-W; NH-$(CH_2)_2$-triazole-$(CH_2)_3$-(polyhydroxylated 5 or 6-cycloalkyl comprising an oxygen atom)-OW; NH-$(CH_2)_2$-triazole-$(CH_2)_3$-(polyhydroxylated 5 or 6-cycloalkyl comprising an oxygen atom)-OH; NH-$(CH_2)_2$-triazole-$(CH_2)_2$-O-($CH_2$-$CH_2$-O$)_3$$(CH_2)_2$-COOH; NH-$(CH_2)_2$-triazole-$(CH_2)_2$-O-($CH_2$-$CH_2$-O$)_3$$(CH_2)_2$-C(O)-W; NH-$(CH_2)_2$-C≡CH; NH-$(CH_2)_2$-$N_3$; NH-$(CH_2)_2$-NH-Q; NH-$(CH_2)_2$-O-($CH_2$-$CH_2$-O$)_x$-$CH_2CH_2$-COOH; NH-$(CH_2)_2$-O-($CH_2$-$CH_2$-O$)_x$-$CH_2CH_2$-NH-Q; NH-$(CH_2)_2$-O-($CH_2$-$CH_2$-O$)_x$-$CH_2CH_2$-CO-NH-W; NH-$(CH_2)_2$-O-($CH_2$-$CH_2$-O$)_x$-$CH_2CH_2$-NH-W; and Q is a protective group;

W is a bioactive compound or a group comprising a bioactive compound.

Preferably, in the compound of formula (I), X and Z are N($CH_3$) or X is S and Z is NH or X is Sand Z is O or X is NH and Z is O or X is NH and Z is NH, or X and Z are O.

In one embodiment, in the compound of formula (I):

X = Z = N(alkyl) with alkyl is linear or branched and comprises from 1 to 5 carbon atoms, preferably $CH_3$;
L is an alkyl group comprising from 2 to 10 carbon atoms
A is a phenyl group.

In another embodiment, in the compound of formula (I):

X=SandZ=NH
A is a phenyl group
L is an alkyl, linear or branched, comprising from 2 to 8 carbon atom; an alkyl, linear or branched, comprising from 2 to 8 carbon atoms and substituted by a COR' group; $(CH_2)_n$-Ph-$(CH_2)_{n'}$ wherein the phenyl is substituted by a COR' group ;
n an integer from 0 to 2
n' an integer from 0 to 2
R' is OH or R;
R is NH-$(CH_2)_r$-triazole-$(CH_2)_2$-O-($CH_2$-$CH_2$-O$)_x$-$CH_2CH_2$C(O)NH-W; NH$(CH_2)_r$-triazole-$(CH_2)_s$-(polyhydroxylated 5 or 6-cycloalkyl comprising an oxygen atom)-OW; NH$(CH_2)_r$-triazole-$(CH_2)_s$-(polyhydroxylated 5 or 6-cycloalkyl comprising an oxygen atom)-OH; NH-$(CH_2)_r$-triazole-$(CH_2)_2$-O-($CH_2$-$CH_2$-O$)_x$$(CH_2)_2$-COOH; NH-$(CH_2)_r$-triazole-$(CH_2)_2$-O-($CH_2$-$CH_2$-O$)_x$$(CH_2)_2$-C(O)-W; NH-alkyl-C=CH wherein the alkyl is linear or branched and comprises

from 2 to 5 carbon atoms; NH-alkyl-$N_3$ wherein the alkyl is linear or branched and comprises from 2 to 5 carbon atoms; NH-alkyl-NH-Q wherein the alkyl is linear or branched and comprises from 2 to 5 carbon atoms and Q is H or a protective group; NH-$(CH_2)_2$-O-$(CH_2$-$CH_2$-O$)_x$-$CH_2CH_2$-COOH; NH-(CHz)z-O-$(CH_2$-$CH_2$-O$)_x$-$CH_2CH_2$-NH-Q; NH-$(CH_2)_2$-O-$(CH_2$-$CH_2$-O$)_x$-$CH_2CH_2$-CO-NH-W;
NH-$(CH_2)_2$-O-$(CH_2$-$CH_2$-O$)_X$-$CH_2CH_2$-NH-W; W; PEG group; a solubilizing group;
r is an integer between 2 to 5,
s is an integer between 2 to 5,
x is an integer between 2 to 8,
W is a bioactive compound or a group comprising a bioactive compound.

Preferably, in this embodiment R is NH-$(CH_2)_2$-triazole-$(CH_2)_2$-O-$(CH_2$-$CH_2$-O$)_3$-$CH_2CH_2$C(O)NH-W; NH-$(CH_2)_2$-triazole-$(CH_2)_3$-(polyhydroxylated 5 or 6-cycloalkyl comprising an oxygen atom)-OW; NH-$(CH_2)_2$-triazole-$(CH_2)_3$-(polyhydroxylated 5 or 6-cycloalkyl comprising an oxygen atom)-OH; NH-(CHz)z-triazole-(CHz)z-O-(CHz-CHz-O$)_3(CH_2)_2$-COOH; NH-$(CH_2)_2$-triazole-$(CH_2)_2$-O-$(CH_2$-$CH_2$-O$)_3(CH_2)_2$-C(O)-W; NH-$(CH_2)_2$-C≡CH; NH-$(CH_2)_2$-$N_3$; NH-$(CH_2)_2$-NH-Q; NH-$(CH_2)_2$-O-$(CH_2$-$CH_2$-O$)_x$-$CH_2CH_2$-COOH; NH-$(CH_2)_2$-O-$(CH_2$-$CH_2$-O$)_x$-$CH_2CH_2$-NH-Q; NH-$(CH_2)_2$-O-$(CH_2$-$CH_2$-O$)_x$-$CH_2CH_2$-CO-NH-W; NH-$(CH_2)_2$-O-$(CH_2$-$CH_2$-O$)_X$-$CH_2CH_2$-NH-W; Q is H or a protective group and W is a bioactive compound or a group comprising a bioactive compound.

In another embodiment, in the compound of formula (I):

X is NH and Z is O
L is $(CH_2)_m$-(triazole)-$(CH_2)_p$-C(O)N$(CH_3)(CH_2)_{m'}$.

In another embodiment in the compound of formula (I):

X and Z are 0
L is $(CH_2)_m$-N$(CH_3)$-C(O)-$(CH_2)_q$-(triazole)-$(CH_2)_p$-C(O)-N$(CH_3)$-$(CH_2)_{m'}$.

In the present invention, the triazole is preferably a 1,2,3-triazole.

Preferably, the compound of formula (I) according to the present invention is a compound chosen among:

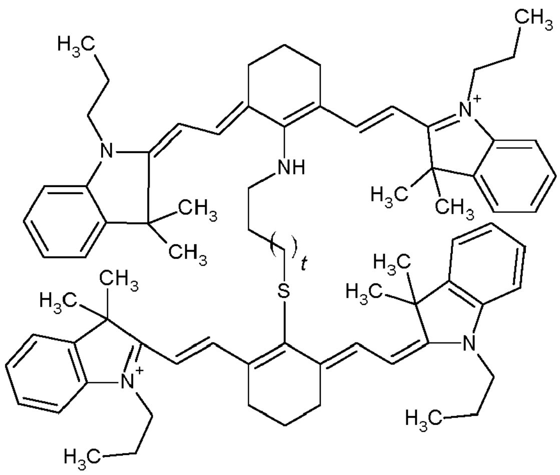

, 2D$^-$,

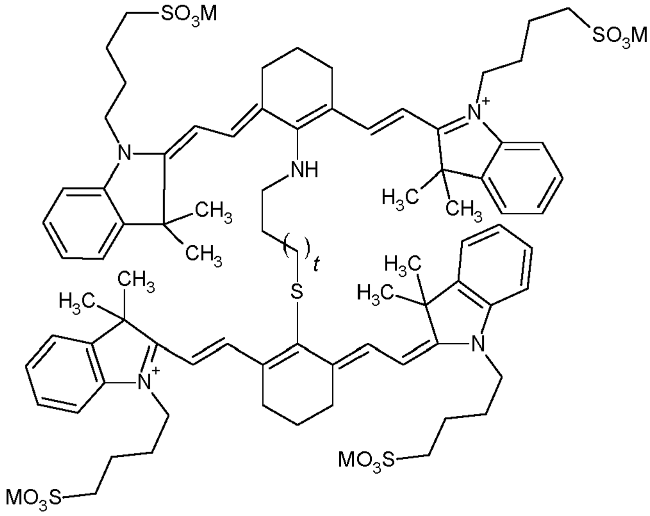

, $2D^{-}$

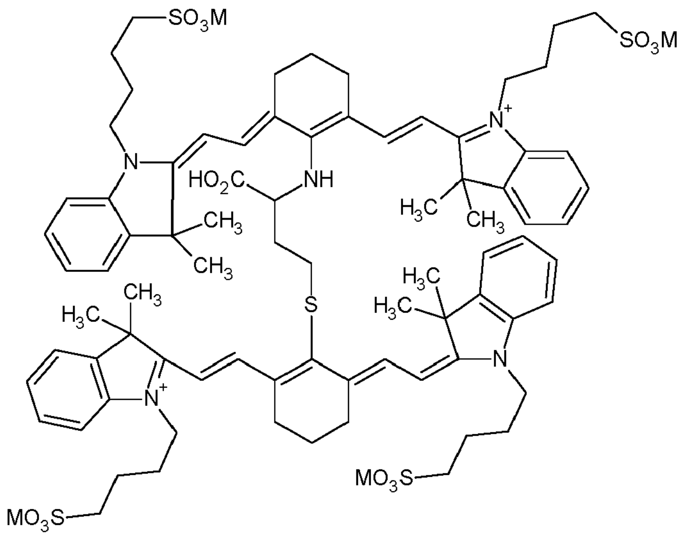

, $2D^{-}$

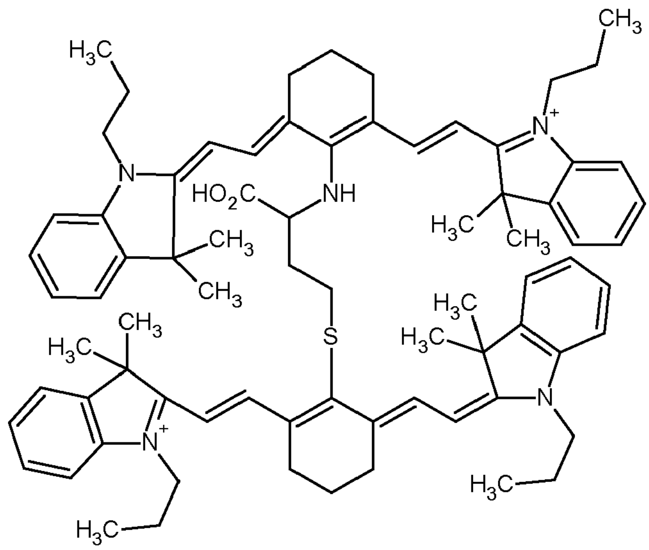

, $2D^{-}$

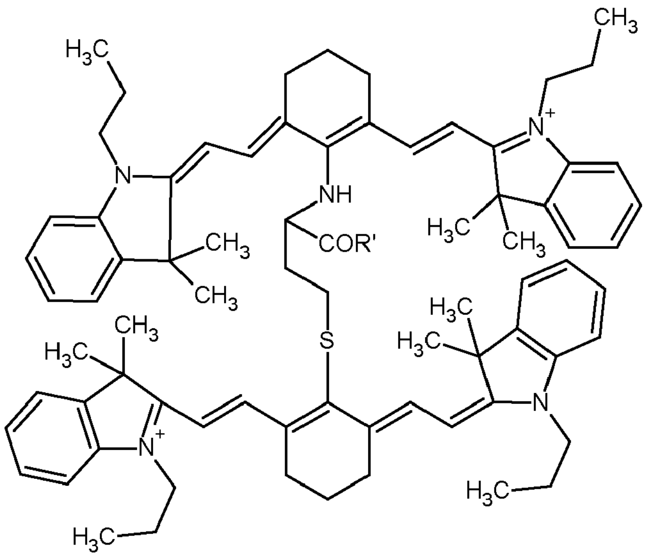

, 2D$^-$

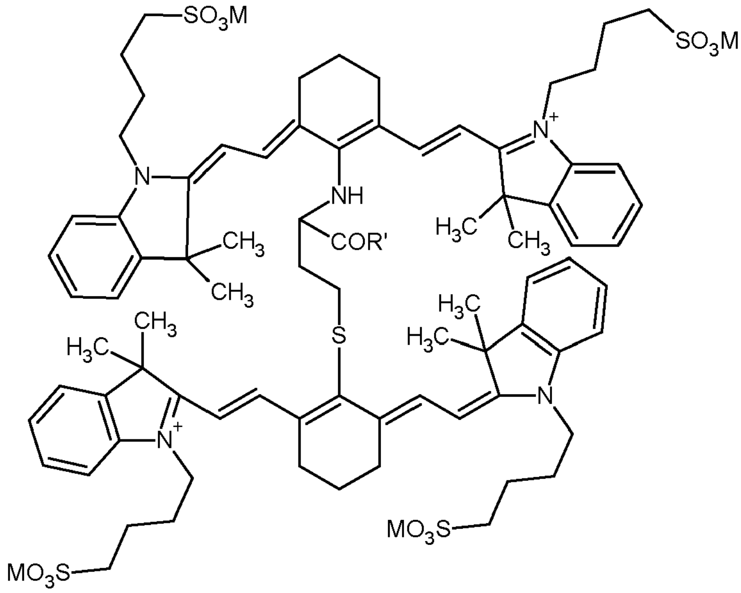

, 2D$^-$

R' is $NH-(CH_2)_{1-3}-(CH_2)-C\equiv CH$, $NH-(CH_2)_{1-3}-(CH_2)-NH-Q$, $NH-(CH_2)_{1-3}-CH_2$-(triazole)-$CH_2-CH_2-O(CH_2-CH_2-O)_{3-7}-CH_2-CH_2-COOH$, $NH-(CH_2)_{1-3}-CH_2$-(triazole)-$CH_2-CH_2-CH_2$-(polyhydroxylated 5 or 6-cycloalkyl comprising an oxygen atom)-OH, $NH-(CH_2)_{1-3}-CH_2$-(triazole)-$CH_2-CH_2-CH_2$-(polyhydroxylated 5 or 6-cycloalkyl comprising an oxygen atom)-OW, $NH-(CH_2)_{1-3}-CH_2$-(triazole)-$CH_2-CH_2-O(CH_2-CH_2-O)_{3-7}-CH_2-CH_2-CONHW$; $NH-(CH_2)_2-O-(CH_2-CH_2-O)_x-CH_2CH_2-COOH$; $NH-(CH_2)_2-O-(CH_2-CH_2-O)_x-CH_2CH_2-NH-Q$; $NH-(CH_2)_2-O-(CH_2-CH_2-O)_x-CH_2CH_2-CO-NH-W$; $NH-(CH_2)_2-O-(CH_2-CH_2-O)_x-CH_2CH_2-NH-W$; wherein W, x are as defined above

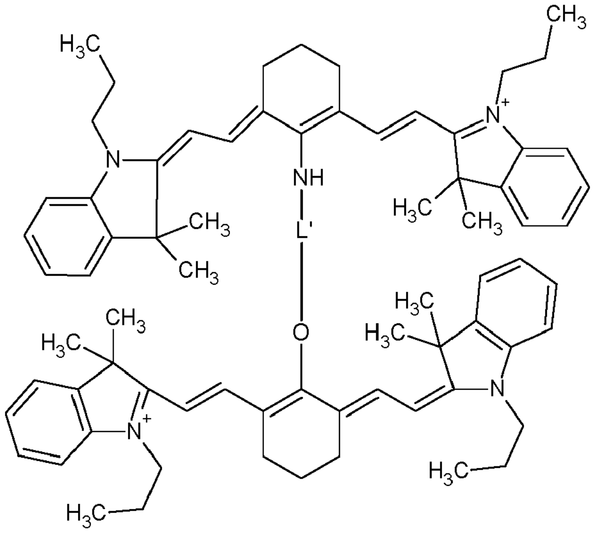

, $2D^-$

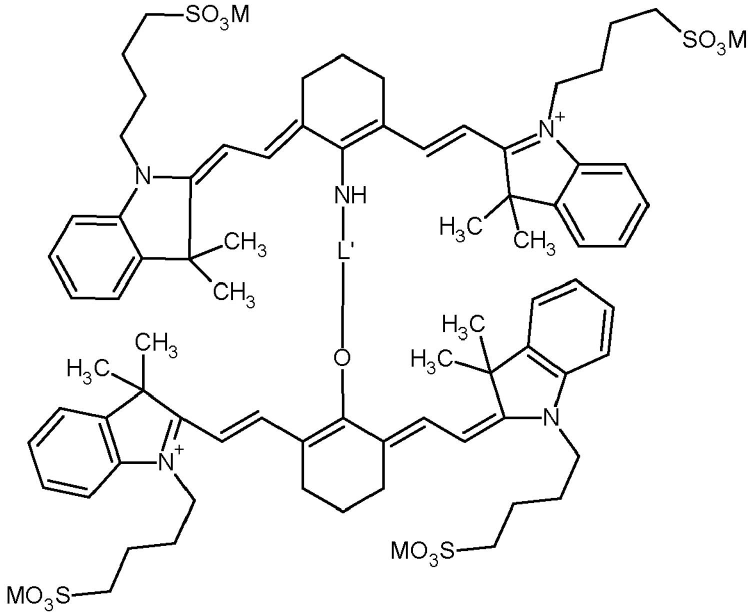

, $2D^-$

L' is $(CH_2)_m$-(triazole)-$(CH_2)_P$-C(O)N($CH_3$)$(CH_2)_{m'}$;
M, p and m' are as defined above

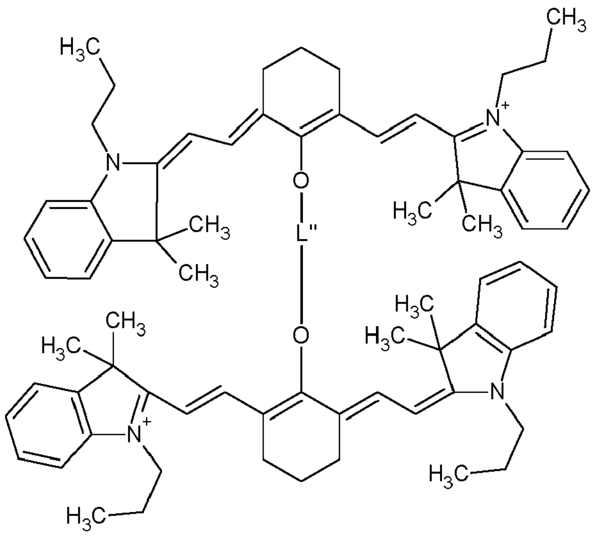

, $2D^-$

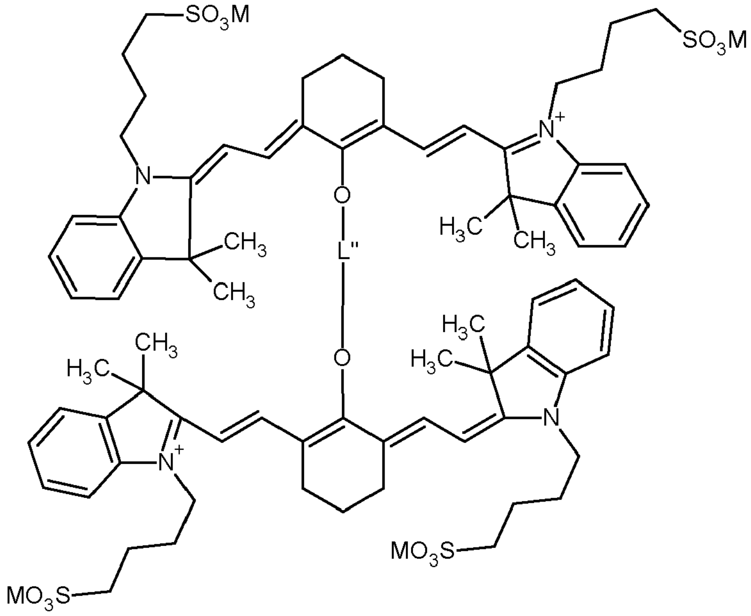

, $2D^-$

L" is $(CH_2)_m$-$N(CH_3)$-C(O)-$(CH_2)_q$-(triazole)-$(CH_2)_p$-C(O)-$N(CH_3)$-$(CH_2)_{m'}$,
M, p, q and m' are as defined above

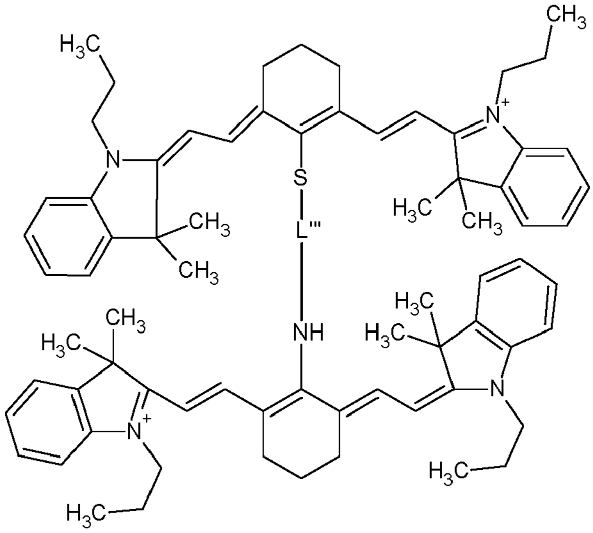

, $2D^-$

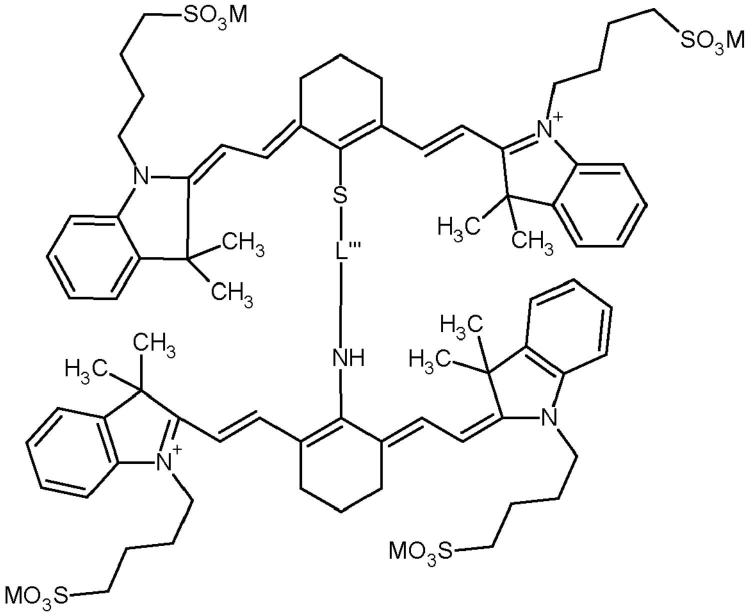

, $2D^-$

L‴ is $(CH_2)_n$-Ph-$(CH_2)_{n'}$, wherein the phenyl is substituted by a COR' group; R' is OH or R, preferably OH; n and n' are as defined above, R is as defined above;

wherein t is an integer from 0 to 6, D each identical or different represents an anion, preferably I; M is H, an ammonium group or a cation, preferably Na or K.

The term "alkyl", as used herein, refers to an aliphatic-hydrocarbon group, which may be straight (or linear) or branched, having from 1 to 10 carbon atoms in the chain unless specified otherwise.

As used herein, the expression "pharmaceutically acceptable salts" refers to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids or aminohydroxyl-O-sulfonic acid; and the like. The pharmaceutically acceptable salts of the present invention can be synthesized from the parent compound which comprises a basic or an acidic moiety, by conventional chemical methods. Furthermore, the expression "pharmaceutically acceptable salt" refers to relatively non-toxic, inorganic and organic acid or base addition salts of the compounds of the present invention. These salts can be prepared in situ during the final isolation and purification of the compounds. In particular, the acid addition salts can be prepared by separately reacting the purified compound in its purified form with an organic or inorganic acid and by isolating the salt thus formed. Among the examples of acid addition salts are the hydrobromide, hydrochloride, hydroiodide, sulfamate, sulfate, bisulfate, phosphate, nitrate, acetate, propionate, succinate, oxalate, valerate, oleate,

palmitate, stearate, laurate, borate, benzoate, lactate, tosylate, citrate, maleate, fumarate, tartrate, naphthylate, mesylate, glucoheptanate, glucoronate, glutamate, lactobionate, malonate, salicylate, methylenebis-b-hydroxynaphthoate, gentisic acid, isethionate, di-p-toluoyltartrate, ethanesulfonate, benzenesulfonate, cyclohexyl sulfamate, quinateslaurylsulfonate salts, and the like. Examples of base addition salts include ammonium salts such as tromethamine, meglumine, epolamine, etc, metal salts such as sodium, lithium, potassium, calcium, zinc or magnesium salts with organic bases such as dicyclohexylamine salts, N-methyl-D-glucamine. Lists of suitable salts may be found in Remington's Pharmaceutical Sciences, 17th ed., Mack Publishing Company, Easton, PA, 1985, p. 1418, P.H. Stahl, C.G. Wermuth, Handbook of Pharmaceutical salts - Properties, Selection and Use, Wiley-VCH, 2002 and S.M. Berge et al. "Pharmaceutical Salts" J. Pharm. Sci, 66: p.1-19 (1977).

Compounds according to the invention also include isotopically-labelled compounds wherein one or more atoms is replaced by an atom having the same atomic number, but an atomic mass or mass number different from the atomic mass or mass number usually found in nature. Examples of isotopes suitable for inclusion in the compounds described above and are not limited to $^{2}H$, $^{3}H$, $^{11}C$, $^{13}C$, $^{14}C$, $^{18}F$, $^{19}F$, $^{13}N$, $^{15}N$, $^{33}S$, $^{34}S$, $^{35}S$, $^{36}S$, $^{17}O$ or $^{18}O$. Isotopically-labelled compounds are useful in drug and/or substrate tissue distribution studies. Substitution with heavier isotopes such as deuterium (2H) affords greater metabolic stability (for example increased in vivo half-life or reduced dosage requirements). Isotopically-labelled compounds are prepared by any suitable method or by processes using an appropriate isotopically-labelled reagent in replacement of the non-labelled reagent otherwise employed.

Advantageously, the compounds according to the invention present:

- significant photothermal activity under 808 nm irradiation for 5 min, with an adequate temperature increase (> 15°C even at low concentration and laser power) which is suitable for PTT application in cancer therapy;
- strong signal (high amplitude) in photoacoustic imaging since these compounds convert absorbed light into heat with good efficiency producing thermal expansion of their surroundings and the production of acoustic waves. A strong signal is defined as equal or better than ICG (Indocyanine green approved by the FDA) in the same conditions;
- good thermal stability toward repeated irradiation over 4 successive heating and cooling cycles due to reduced photobleaching.

The compounds of the invention, comprising a COOH group can advantageously be modified by grafting a solubilizing group, a specific ligand to target a microbial agent, a cell or a tumor.

The invention also relates to a composition comprising at least one compound of formula (I).

The composition according to the invention preferably comprises at least one pharmaceutically acceptable excipient.

The expression "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The term "pharmaceutically acceptable carrier" or "pharmaceutically acceptable excipient" is employed for any excipient, solvent, dispersion medium, absorption retardant, diluent or adjuvant etc. , such as preserving or antioxidant agents, fillers, binders, disintegrating agents, wetting agents, emulsifying agents, suspending agents, solvents, dispersion media, coatings, antibacterial agents, isotonic and absorption delaying agents and the like, that does not produce a secondary reaction, for example an allergic reaction, in humans or animals. Typical, non-limiting examples of excipients include mannitol, lactose, magnesium stearate, sodium saccharide, talcum, cellulose, sodium crosscarmellose, glucose, gelatine, starch, lactose, dicalcium phosphate, sucrose, kaolin, magnesium carbonate, wetting agents, emulsifying agents, solubilizing agents, sterile water, saline, pH buffers, nonionic surfactants, lubricants, stabilizing agents, binding agents and edible oils such as peanut oil, sesame oils and the like. In addition, various excipients commonly used in the art may be included. Pharmaceutically acceptable carriers or excipients are well known to a person skilled in the art, and include those described in Remington's Pharmaceutical Sciences (Mack Publishing Company, Easton, USA, 1985), Merck Index (Merck & Company, Rahway, N.J.), Gilman et al (Eds. The pharmacological basis of therapeutics, 8th Ed. , Pergamon press. , 1990). Except insofar as any conventional media or adjuvant is incompatible with the active ingredient according to the invention, its use in the therapeutic compositions is contemplated.

The present invention also refers to a compound or a composition according to the invention for its use as a medicine, preferably for treatment of microbial infection or of cancer, preferably of cancer.

The present invention also refers to a compound or a composition according to the invention for its use for the preparation of a medicine, preferably for the preparation of a medicine for the treatment of microbial infection or of cancer, preferably of cancer.

The present invention also refers to the use of a compound or a composition according to the invention for the treatment of microbial infection or of cancer, preferably cancer.

The compounds according to the invention can be used for diagnosis by fluorescent imaging and/or photoacoustic imaging. Preferably for diagnosis of tumor.

The present invention also refers to a compound or a composition according to the invention for its use as a diagnosis agent for fluorescent imaging and/or photoacoustic imaging. Preferably for diagnosis of tumor.

The present invention also refers to a compound or a composition according to the invention for its use for the preparation of a diagnosis agent for fluorescent imaging and/or photoacoustic imaging. Preferably for diagnosis of tumor.

The present invention also refers to the use of a compound or a composition according to the invention as a diagnosis agent for fluorescent imaging and/or photoacoustic imaging. Preferably for diagnosis of tumor.

Advantageously, the compounds of the invention present a high thermal conversion coefficient and can thus be used for photothermal treatment guided by fluorescence and/or photoacoustic imaging, advantageously guided by fluorescence and photoacoustic imaging. The compounds of the present invention can be used for photothermal treatment guided by fluorescence and/or photoacoustic imaging, advantageously guided by fluorescence and photoacoustic imaging of cancer or microbial infection, preferably cancer.

Also described is a method for the treatment of microbial infections or cancer, preferably cancer comprising the administration of a therapeutically effective amount of a compound selected within the compounds of formula (I) according to the invention, or of a pharmaceutical composition according to the invention to a patient in need thereof.

Also described is a method for the diagnosis of tumor, preferably cancer comprising the administration of a therapeutically effective amount of a compound selected within the compounds of formula (I) according to the invention, or of a pharmaceutical composition according to the invention to a patient in need thereof.

The term "patient" means a person or an animal at risk of having tumors or, a person or an animal having a microbial infection or a cancer. As used herein, the term "patient" refers to a warm-blooded person or animal such as a mammal, preferably a human or a human child, who is afflicted with, or has the potential to be afflicted with one or more infections and conditions described herein. The identification of those subjects who are in need of treatment of herein-described diseases and conditions is well within the ability and knowledge of one skilled in the art. A veterinarian or a physician skilled in the art can readily identify, by the use of clinical tests, physical examination, medical or family history or biological and diagnostic tests, those subjects who are in need of such a treatment.

The expression "therapeutically effective amount" or "pharmaceutically effective amount" as used herein, refer to an amount of a compound according to the invention, which when administered to a patient in need thereof, is sufficient to effect treatment for disease-states, conditions, or disorders for which the compound has utility. Such an amount would be sufficient to elicit the biological or medical response of a tissue system, or patient that is sought by a researcher or a clinician.

The compound according to the invention may be for example provided under the form of an injectable product or under the form of a patch..

The compounds according to the present invention are more specifically useful for Glioblastoma, prostate cancer, breast cancer and melanoma.

The terms "treatment", "treat" and "treating" as used herein are intended to mean in particular the administration of a treatment comprising a compound or composition according to the invention to a patient suffering from an infection.

The invention is now described with respect to the following non limiting examples.

## Examples

### Protocol synthesis and physico-chemical characterization

**N-2-S Heterodimer**

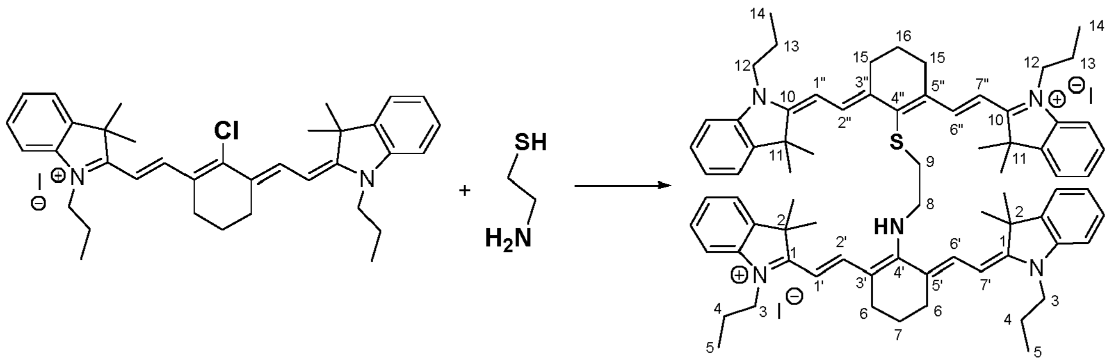

To a solution of 87 mg of commercial IR780 (0.13 mmol, 2 eq.) in freshly distilled acetonitrile (1 mL) were added 18 mg of potassium carbonate (0.13 mmol, 2.0 eq.) and 5.01 mg of 2-amino ethanethiol (0.065 mmol, 1 eq.) at room temperature under inert atmosphere. The solution was stirred for 18 hours at room temperature then the solvent was removed under vacuum. The crude product was purified by flash chromatography (DCM/AcOEt 50/50) to give the product as a blue gum (38 mg, 43%). 1H NMR (400 MHz, CDCl3): δ 0.96 (t, 12H, J = 7.5 Hz, H-5 and H-14), 1.62 (s, 12H, CH3), 1.70 (s, 12H, CH3), 1.76-1.87 (m, 12H, H-7, H-4, H-16, H-13), 2.41 (m, 4H, H-6), 2.60 (m, 4H, H-15), 3.28-3.35 (m, 2H, H-9), 3.79 (bt, 4H, $J_{3,4}$ = 6.5 Hz, H-3), 3.86-3.93 (m, 2H, H-8), 4.04 (bt, 4H, $J_{12,13}$ = 6.5 Hz, H-12), 5.64 (d, 2H, J = 13.0 Hz, H-1', H-7'), 6.15 (d, 2H, J = 13.5 Hz, H-1", H-7"), 6.85 (d, 2H, J = 8.0 Hz, HAr), 7.03 (t, 2H, J = 8.0 Hz, HAr), 7.07 (d, 2H, J = 8.0 Hz, HAr), 7.16-7.28 (m, 6H, HAr), 7.32-7.39 (m, 4H, HAr), 7.67 (d,, 2H, H-2', H-6'), 8.22 (bs, 1H, NH), 8.68 (d, 2H, H-2", H-6"). 13C NMR (100.6 MHz, CDCl3): δ 11.8 (2C-5 or 2C-14), 11.9 (2C-5 or 2C-14), 20.3 (2C-4), 20.9 (C-16), 21.0 (2C-13), 21.8 (C-7), 25.3 (2C-6), 26.8 (2C-15), 28.4 (4CH3), 29.2 (4CH3), 37.3 (C-9), 45.1 (2C-3), 46.3 (2C-12), 48.0 (2C-2), 49.4 (C-8), 49.6 (2C-11) 95.1 (C-1' and C-7'), 101.5 (C-1" and C-7"), 108.9 (2CAr), 110.7 (2CAr), 121.1 (C-3' and C-5'), 122.0 (2CAr), 122.5 (2CAr), 122.9 (2CAr), 125.2 (2CAr), 128.2 (2CAr), 128.8 (2CAr), 133.9 (C-3" and C-5"), 138.5 (C-2' and C-6'), 140.4 (2CqAr), 141.1 (2CqAr), 142.5 (2CqAr), 143.2 (2CqAr), 145.4 (C-2" and C-6"), 156.7 (C-4"), 167.9 (2C-1), 169.0 (C-4'), 172.3 (2C-10). HRMS (ESI): [$M^{2+}$] calcd for [$C_{74}H_{93}N_5S^{2+}$] 541.8570, found 541.8557. IR ($\overline{\upsilon}$ (cm-1)): 2963, 2927, 1537, 1516, 1452, 1367, 1247, 1230, 1163, 1153, 1056, 929, 796.

**N-6-S heterodimer**

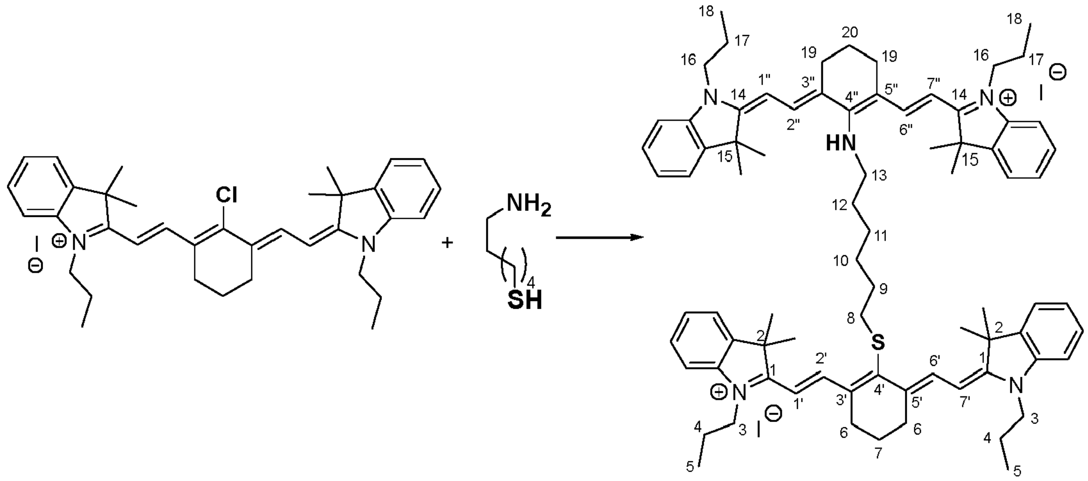

To a solution of 115 mg of commercial IR780 (0.17 mmol, 2 eq.) in freshly distilled acetonitrile (2 mL) were added 42 mg of potassium carbonate (0.303 mmol, 3.5 eq.) and 14.7 mg of 6-amino hexanethiol (0.086 mmol, 1 eq.) at room temperature under inert atmosphere. The solution was stirred for 48 hours at room temperature then the solvent was removed under *vacuum.* The crude product was purified by flash chromatography on silica gel (DCM/MeOH 95/5) to give the product as a blue gum (47 mg, 45%).

$^{1}$H NMR (400 MHz, CDCl3): δ 0.99 (t, 6H, $J_{18,17}$ = 7.5 Hz, H-18), 1.05 (t, 6H, $J_{5,4}$= 7.5 Hz, H-5), 1.29-1.37 (m, 2H, H-11), 1.42-1.49 (m, 2H, H-10), 1.58-1.68 (m, 2H, H-9), 1.64 (s, 12H, $CH_3$), 1.72 (s, 12H, $CH_3$), 1.73-1.81 (m, 6H, H-17, H-20), 1.84-1.98 (m, 8H, H-7, H-4, H-12), 2.43 (t, 4H, $J_{19,20}$ = 6.5 Hz, H-19), 2.63 (bt, 4H, $J_{6,7}$ = 6.5 Hz, H-6), 2.75 (t, 2H, $J_{8,9}$

= 7.5 Hz, H-8), 3.69-3.84 (m, 6H, H-13 and H-16), 4.07 (bt, 4H, $J_{3,4}$ = 7.5 Hz, H-3), 5.55 (d, 2H, $J$ = 13.0 Hz, H-1", H-7"), 6.16 (d, 2H, $J$ = 14.0 Hz, H-1', H-7'), 6.81 (d, 2H, $J$ = 8.0 Hz, HAr), 6.99 (t, 2H, $J$ = 8.0 Hz, HAr), 7.10 (d, 2H, $J$ = 8.0 Hz, HAr), 7.15-7.25 (m, 4H, HAr), 7.34 (t, 2H, $J$ = 8.0 Hz, HAr), 7.42 (d, 2H, $J$ = 8.0 Hz, HAr), 7.64 (d, 2H, H-2", H-6"), 8.46 (bs, 1H, NH), 8.80 (d, 2H, H-2', H-6'). $^{13}C$ NMR (100.6 MHz, CDCl3): δ 11.9 (2C-5), 11.9 (2C-18), 20.1 (2C-17,) 20.9 (2C-4), 21.0 (C-7), 21.6 (C-20), 25.5 (2C-19), 26.6 (C-11), 26.6 (2C-6), 28.2 (4CH3), 28.8 (C-10), 29.3 (4$CH_3$), 30.6 (C-9), 31.3 (C-12), 38.3 (C-8), 44.9 (C-13), 46.3 (2C-3), 47.7 (2C-15), 49.4 (2C-2), 50.1 (2C-16), 94.0 (C-1" and C-7"), 101.2 (C-1' and C-7'), 108.4 (2CAr), 110.7 (2CAr), 120.3 (C-3" and C-5"), 122.0 (2CAr), 122.4 (2CAr), 122.6 (2CAr), 125.2 (2CAr), 128.1 (2CAr), 128.7 (2CAr), 133.9 (C-3' and C-5'), 137.6 (C-2" and C-6"), 140.1 (2CqAr), 141.0 (2CqAr), 142.5 (2CqAr), 143.4 (2CqAr), 146.0 (C-2' and C-6'), 158.6 (C-4'), 166.8 (2C-14), 169.6 (C-4"), 172.3 (2C-1). HRMS (ESI): [$M^{2+}$] calcd for [$C_{78}H_{101}N_5S^{2+}$] 569.8884, found 569.8875. IR ($\bar{\upsilon}$ (cm-1)): 2964, 2929, 1535, 1515, 1450, 1365, 1247, 1230.

**N-3-S-COOH heterodimer**

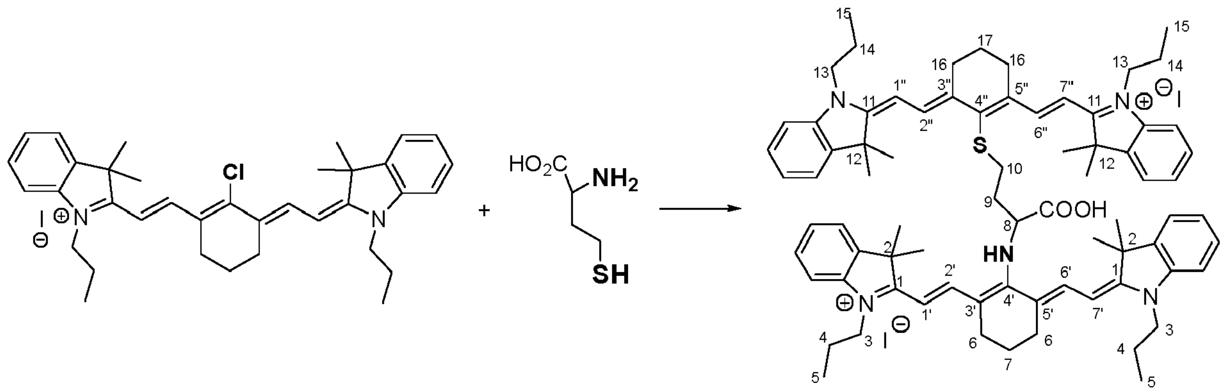

To a solution of 40 mg of commercial IR780 (0.059 mmol, 2 eq.) in freshly distilled dimethylformamide (0.5 mL) were added 18 mg of triethylamine (0.089 mmol, 3 eq.) and 4.0 mg of L-homocysteine (0.029 mmol, 1 eq.) at room temperature under inert atmosphere. The solution was stirred for 18 hours at room temperature then the solvent was removed under *vacuum.* The crude product was purified by flash chromatography on silica gel (DCM/MeOH 95/5) to give the product as a blue gum (28 mg, 70%).

$^{1}H$ NMR (400 MHz, $CDCl_3$): δ 0.95 (t, 6H, $J$ = 7.5 Hz, H-5 or H-15), 0.98 (t, 6H, $J$ = 7.5 Hz, H-5 or H-15), 1.58 (s, 6H, $CH_3$), 1.62 (s, 6H, $CH_3$), 1.63 (s, 6H, $CH_3$), 1.68 (s, 6H, $CH_3$), 1.76-1.87 (m, 12H, H-7, H-4, H-17, H-14), 2.22-2.35 (m, 3H, 2H-6 or 2H-16 and H-9a), 2.44-2.55 (m, 3H, 2H-6 or 2H-16 and H-9b), 2.60-2.67 (m, 5H, 4H-6 or 4H-16 and H-10a), 2.80 (app dd, 1H, $J_{10a,10b}$ = 12 Hz, $J_{10b,9a}$ = 4.5 Hz, H-10b), 3.70-3.80 (m, 4H, H-3 or H-13), 3.99-4.14 (m, 4H, H-3 or H-13), 4.33-4.38 (m, 1H, H-8), 5.62 (d, 2H, $J$ = 13.0 Hz, H-1', H-7'), 6.17 (d, 2H, $J$ = 13.5 Hz, H-1", H-7"), 6.85 (d, 2H, $J$ = 8.0 Hz, HAr), 7.04 (t, 2H, $J$ = 8.0 Hz, HAr), 7.07 (d, 2H, $J$ = 8.0 Hz, HAr), 7.16-7.26 (m, 6H, HAr), 7.30 (t, 2H, $J$ = 8.0 Hz, HAr), 7.35 (t, 2H, $J$ = 8.0 Hz, HAr), 7.80 (d, 2H, H-2', H-6'), 8.79 (d, 2H, H-2", H-6"), 9.10 (bs, 1H, NH). $^{13}C$ NMR (100.6 MHz, CDCl3): δ 11.8 (2C-5 or 2C-15), 11.9 (2C-5 or 2C-15), 20.3 (2C-4), 21.0 (C-17), 21.1 (2C-14), 21.9 (C-7), 25.6 (2C-6 or 2C-16), 26.9 (2C-6 or 2C-16), 28.2 (4$CH_3$), 28.4 (4$CH_3$), 29.7 (C-9), 33.2 (C-10), 44.9 (2C-3 or 2C-13), 46.3 (2C-3 or 2C-13), 47.9 (2C-2), 49.4 (2C-12), 60.7 (C-8), 94.8 (C-1' and C-7'), 101.4 (C-1" and C-7"), 108.8 (2CAr), 110.7 (2CAr), 120.6 (C-3' and C-5'), 122.2 (2CAr), 122.5 (2CAr), 122.9 (2CAr), 125.1 (2CAr), 128.3 (2CAr), 128.7 (2CAr), 134.6 (C-3" and C-5"), 137.9 (C-2' and C-6'), 140.4 (2CqAr), 141.1 (2CqAr), 142.6 (2CqAr), 143.3 (2CqAr), 146.3 (C-2" and C-6"), 156.7 (C-4"), 165.8 (2C-1), 167.8 (C-4'), 171.9 (C=O), 172.4 (2C-11). HRMS (ESI): [$M^{2+}$] calcd for [$C_{76}H_{95}N_5O_2S^{2+}$] 570.8598, found 570.8588.

**N-3-S-alkyne heterodimer**

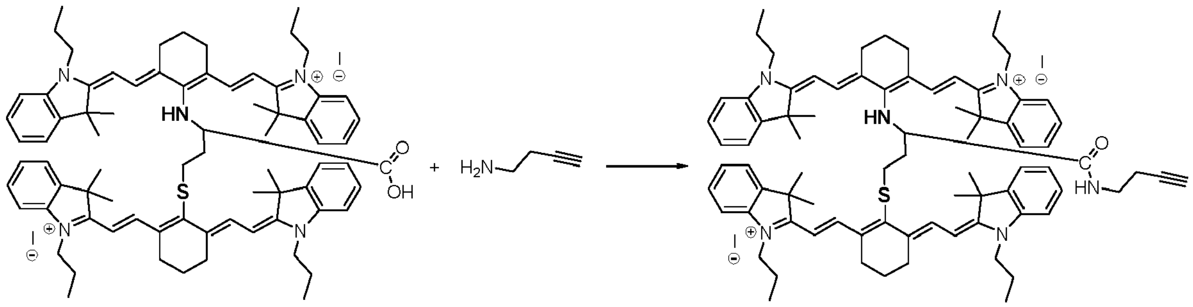

To a solution of 43 mg of N-3-S-COOH heterodimer (0.031 mmol, 1 eq.) in freshly distilled acetonitrile (3 mL) were added 14 mg of HATU (0.037 mmol, 1.2 eq.), 4 mg of 4-aminobutyne (0.037 mmol, 1.2 eq.) and 13 μl of DIPEA (0.074 mmol, 2.4 eq.) at room temperature under inert atmosphere. The solution was stirred for 3 hours at room temperature. The mixture was diluted with 30 mL of dichloromethane and quenched with 30 mL of saturated aqueous solution of $NH_4Cl$, then the aqueous layer was extracted with dichloromethane (2 × 30 mL). The organic combined layer was dried over $MgSO_4$, and the solvent was removed under vacuum. The crude product was purified by flash chromatography on silica gel (eluent: DCM/MeOH 100/0 to 95/5) to afford the compound as a green/blue gum (26mg, 59%).

$^1$H NMR (400 MHz, CDCl3): δ 0.97 (t, 6H, *J* = 7.5 Hz, H-5 or H-15), 1.00 (t, 6H, *J* = 7.5 Hz, H-5 or H-15), 1.63 (s, 6H, $CH_3$), 1.66 (s, 6H, $CH_3$), 1.70 (s, 6H, $CH_3$), 1.71 (s, 6H, $CH_3$), 1.74-1.90 (m, 10H, H-7 or H-17, H-4, H-14), 1.88 (t, 1H, $J_{21,19}$ = 2.5 Hz, H-21), 1.94 (t, 2H, *J* = 6.0 Hz, H-7 or H-17), 2.10-2.21 (m, 1H, H-9a), 2.26-2.36 (m, 1H, H-9b), 2.34 (bt, 2H, *J* = 7.5 Hz, H-19), 2.41 (bt, 4H, *J* = 7.5 Hz, H-6 or H-16), 2.51-2.70 (m, 5H, H-6 or H-16 and H-10a), 2.79 (app dd, 1H, $J_{10a,10b}$ = 12.0 Hz, $J_{10b,9a}$, = 4.5 Hz, H-10b), 3.37-3.48 (m, 2H, H-18), 3.70-3.80 (m, 4H, H-3 or H-13), 3.97 (bt, 4H, *J* = 7.5 Hz, H-3 or H-13), 4.18-4.25 (m, 1H, H-8), 5.81 (d, 2H, *J* = 13.0 Hz, H-1', H-7'), 6.14 (d, 2H, *J* = 13.5 Hz, H-1", H-7"), 6.93 (d, 2H, *J* = 8.0 Hz, HAr), 6.99 (bt, 1H, $J_{NH,18}$ = 6.0 Hz, NH), 7.05 (t, 2H, *J* = 8.0 Hz, HAr), 7.13 (d, 2H, *J* = 8.0 Hz, HAr), 7.20 (t, 2H, *J* = 8.0 Hz, HAr), 7.26-7.37 (m, 8H, HAr), 7.85 (d, 2H, H-2', H-6'), 8.79 (d, 2H, H-2", H-6"). $^{13}$C NMR (100.6 MHz, $CDCl_3$): δ 11.6 (2C-5 or 2C-15), 11.7 (2C-5 or 2C-15), 19.4 (C-19), 20.4 (2C-4), 20.9 (C-17), 20.9 (2C-14), 21.6 (C-7), 24.9 (2C-6 or 2C-16), 26.4 (2C-6 or 2C-16), 28.0 ($2CH_3$), 28.1 ($2CH_3$), 28.2 ($2CH_3$), 28.8 ($2CH_3$), 32.8 (C-10), 35.1 (C-9), 38.7 (C-18), 45.3 (2C-3 or 2C-13), 46.0 (2C-3 or 2C-13), 48.4 (2C-2), 49.4 (2C-12), 61.5 (C-8), 69.6 (C-21), 81.6 (C-20), 97.8 (C-1' and C-7'), 101.3 (C-1" and C-7"), 109.6 (2CAr), 110.6 (2CAr), 122.2 (2CAr), 122.5 (2CAr), 122.8 (2CAr), 123.9 (C-3' and C-5'), 125.2 (2CAr), 128.4 (2CAr), 128.7 (2CAr), 134.3 (C-3" and C-5"), 140.1 (C-2' and C-6'), 140.7 (2CqAr), 141.1 (2CqAr), 142.5 (2CqAr), 143.0 (2CqAr), 146.1 (C-2" and C-6"), 156.6 (C-4"), 164.6 (2C-1), 165.9 (C-4'), 170.1 (C=O), 172.6 (2C-11). HRMS (ESI): [$M^{2+}$] calcd for [$C_{80}H_{100}N_6OS^{2+}$] 596.3834, found 596.3837.

## N-3-S-PEG heterodimer

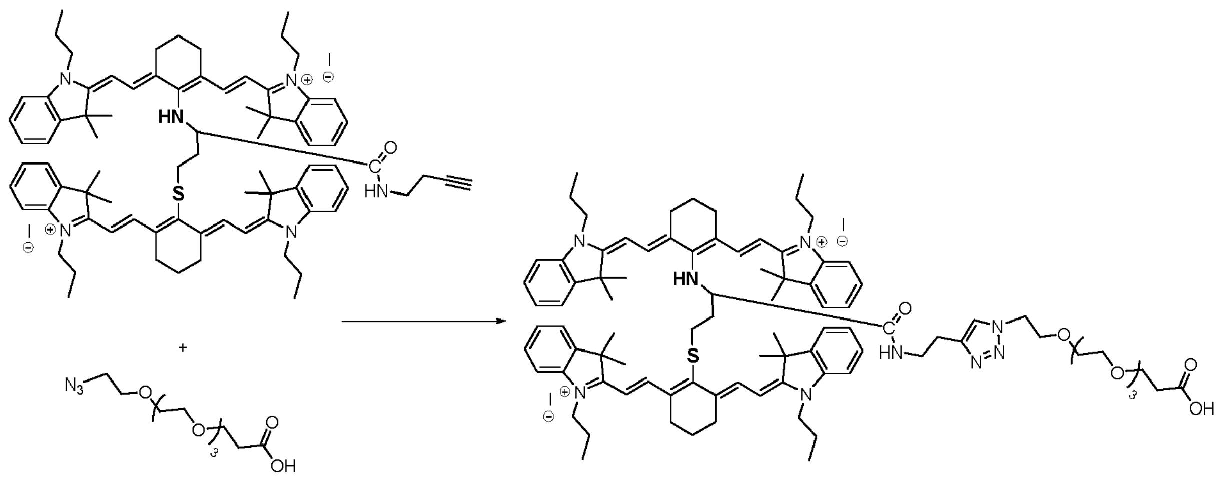

To a solution of N-3-S-alkyne (101.3 mg, 70 μmol, 1.0 eq.) in a mixture of THF/$H_2O$ (2/1 v/v) (6 mL), 20 mg of azido-dPEG®4-acid (70 μmol, 1.0 eq.), 8.8 mg of copper (II) sulphate (35 μmol, 0.5 eq.), and 13.7 mg of sodium ascorbate (70 μmol, 1 eq.) were added, and the mixture was stirred at room temperature for 48 h. The solvent was removed under *vacuum* and the residue was diluted in dichloromethane (20 mL) and washed with water (20 mL). The organic layer was

dried over $MgSO_4$, and the solvent was removed under vacuum. The crude product was purified by flash chromatography on silica gel (eluent: DCM/MeOH 100/0 to 95/5) to afford the compound as a green gum (55 mg, 46%).

$^{1}$H NMR (400 MHz, CDCl3): δ 0.97 (t, 6H, *J* = 7.5 Hz, H-5 or H-15), 0.99 (t, 6H, *J* = 7.5 Hz, H-5 or H-15), 1.62 (s, 6H, $CH_3$), 1.64 (s, 6H, $CH_3$), 1.70 (s, 12H, $CH_3$), 1.72-1.87 (m, 10H, H-7 or H-17, H-4, H-14), 1.93 (t, 2H, *J* = 6.0 Hz, H-7 or H-17), 2.09-2.19 (m, 1H, H-9a), 2.22-2.46 (m, 5H, H-9b and H-6 or H-16), 2.50-2.69 (m, 7H, H-6 or H-16, H-10a and H-29), 2.73-2.90 (m, 3H, H10b and H-19), 3.37-3.47 (m, 1H, H-18a), 3.53-3.71 (m, 15H, H-18b, H-22, H-23, H-24, H-25, H-26, H-27, H-28), 3.78-3.90 (m, 6H, H-3 or H-13 and H-21), 3.96 (t, 4H, J = 7.5 Hz, H-3 or H-13), 4.10-4.18 (m, 1H, H-8), 4.51-4.58 (m, 2H, H-20), 5.66 (bs, 1H, NH), 5.82 (d, 2H, *J* = 13.0 Hz, H-1', H-7'), 6.14 (d, 2H, *J* = 13.5 Hz, H-1", H-7"), 6.91 (bs, 1H, NH), 6.93 (d, 2H, *J* = 8.0 Hz, HAr), 7.06 (d, 2H, *J* = 8.0 Hz, HAr), 7.13 (t, 2H, *J* = 8.0 Hz, HAr), 7.20 (t, 2H, *J* = 8.0 Hz, HAr), 7.26-7.38 (m, 8H, HAr), 7.64 (s, 1H, H-triazole), 7.84 (d, 2H, H-2', H-6'), 8.78 (d, 2H, H-2", H-6"). $^{13}$C NMR (100.6 MHz, CDCl3): δ 11.6 (2C-5 or 2C-15), 11.7 (2C-5 or 2C-15), 20.4 (2C-4), 20.9 (2C-14), 21.6 (C-17), 22.8 (C-7), 24.9 (2C-6 or 2C-16), 25.7 (C-19), 26.4 (2C-6 or 2C-16), 28.1 ($2CH_3$), 28.2 ($2CH_3$), 28.2 ($2CH_3$), 28.7 ($2CH_3$), 33.0 (C-10), 34.1 (C-29), 35.3 (C-9), 39.2 (C-18), 45.3 (2C-3 or 2C-13), 45.9 (2C-3 or 2C-13), 48.5 (2C-2), 49.4 (2C-12), 49.6 (C-20), 61.7 (C-8), 69.7, 69.7, 69.8, 69.9, 69.9, 70.0, 70.1, 70.2 (C-21, C-22, C-23, C-24, C-25, C-26, C-27, C-28), 97.9 (C-1' and C-7'), 101.3 (C-1" and C-7"), 109.6 (2CAr), 110.7 (2CAr), 122.2 (2CAr), 122.5 (2CAr), 122.9 (2CAr), 123.2 (CH-triazole), 124.0 (C-3' and C-5'), 125.2 (2CAr), 128.4 (2CAr), 128.8 (2CAr), 134.3 (C-3" and C-5"), 140.2 (C-2' and C-6'), 140.7 (2CqAr), 141.1 (2CqAr), 142.5 (2CqAr), 143.0 (2CqAr), 145.1 (C-triazole), 146.0 (C-2" and C-6"), 156.7 (C-4"), 164.5 (2C-1, C4'), 170.2 (C=O), 172.5 (2C-11), 177.6 (C=O). HRMS (ESI): [$M^{2+}$] calcd for [$C_{91}H_{121}N_9O_7S^{2+}$] 741.9549, found 741.9559.

**N-3-S-glucose Heterodimer**

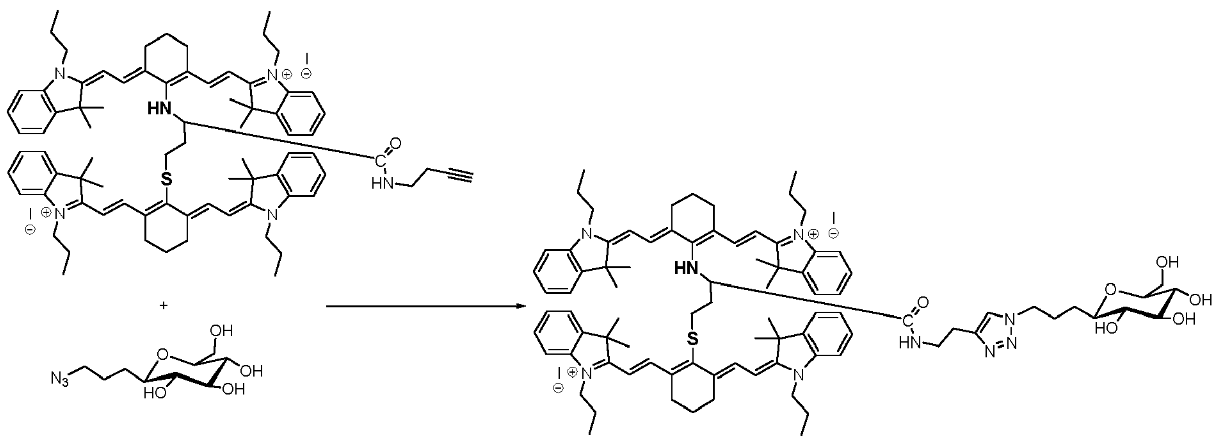

To a solution of N-3-S-alkyne (63 mg, 43 μmol, 1.0 eq.) in a mixture of THF/$H_2O$ (2/1 v/v) (6 mL), 17.3 mg of azido-D-glucopyranose derivative (65 μmol, 1.5 eq.), 10.7 mg of copper (II) sulphate (43 μmol, 1 eq.), and 17 mg of sodium ascorbate (86 μmol, 2 eq.) were added, and the mixture was stirred at 40°C for 48 h in a sealed tube. The solvent was removed under *vacuum* and the residue was diluted in dichloromethane (10 mL) and washed with water (10 mL). The organic layer was dried over $MgSO_4$, and the solvent was removed under vacuum. The crude product was purified by preparative HPLC with a C18 column (VKR5 C18 22x250 mm, the gradient mobile phase was A: 99.08/0.02 water/TFA, B: 99.08/0.02 ACN/TFA; 40 to 100%B; flow rate 23 mL/min) to afford the compound as a green foam after evaporation under *vacuum* and lyophilization (14 mg, 20%).

1H NMR (400 MHz, DMSO-D6): δ 0.87 (t, 6H, J = 7.5 Hz, H-5 or H-15), 0.89 (t, 6H, J = 7.5 Hz, H-5 or H-15), 1.55 (s, 6H, CH3), 1.57 (s, 6H, CH3), 1.59-1.66 (m, 5H, H-4 or H14, H21a), 1.65 (s, 6H, CH3), 1.68 (s, 6H, CH3), 1.68-1.79 (m, 7H, H-4 or H-14, H22a, H-7 or H-17), 1.80-1.87 (m, 3H, H-7 or H-17, H22b), 1.93-2.01 (m, 1H, H21b), 2.07-2.19 (m, 1H, H-9a), 2.25-2.35 (m, 1H, H-9b), 2.36-2.47 (m, 4H, H-6 or H-16), 2.60-2.70 (m, 4H, H-6 or H-16), 2.70-2.79 (m, 4H, H-10, H-19), 2.83 (app t, 1H, $J_{25,24}$ = $J_{25,26}$ = 9.0 Hz, H-25), 2.95-3.02 (m, 2H, H-26, H-27), 3.10 (app t, 1H, $J_{24,23}$ = $J_{24,25}$ = 9.0 Hz, H-24), 3.31-3.40 (m, 4H, H-18, H-23, , H-28a), 3.65 (bd, 1H, $J_{28a,28b}$ = 12.0 Hz, H-28b), 3.94 (bt, 4H, J = 7.0 Hz, H-3 or H-13), 4.11 (bt, 4H, J = 7.0 Hz, H-3 or H-13), 4.24-4.31 (m, 3H, H-8, H-20), 5.87 (d, 2H, J = 13.0 Hz, H-1', H-7'), 6.30 (d, 2H, J = 13.5 Hz, H-1", H-7"), 7.13 (t, 2H, J = 8.0 Hz, HAr), 7.23 (d, 2H, J = 8.0 Hz, HAr), 7.23-7.29 (m, 2H, HAr), 7.33 (t, 2H, J = 8.0 Hz, HAr), 7.37-7.45 (m, 6H, HAr), 7.58 (d, 2H, J = 8.0 Hz, HAr), 7.76 (d, 2H, H-2', H-6'), 7.84 (s, 1H, H-triazole), 8.56 (bs, 1H, NH), 8.72 (d, 2H, H-2", H-6"). 13C NMR (100.6 MHz, DMSO-D6): δ 11.0 (2C-5 or 2C-15), 11.1 (2C-5 or 2C-15), 19.8 (2C-4), 20.4 (2C-14 and C-17), 21.5 (C-7), 24.4 (2C-6 or 2C-16), 25.5 (C-19), 25.8 (C-22), 26.4 (2C-6 or 2C-16), 27.5 (2CH3), 27.6 (2CH3), 27.8 (2CH3), 27.9 (2CH3), 28.4 (C-9), 29.0 (C-21), 31.6, (C-10), 38.7 (C-18), 44.0 (2C-3 or 2C-13), 44.9 (2C-3 or

2C-13), 47.7 (2C-2), 48.8 (2C-12), 49.5 (C-20), 61.5 (C-8), 65.8 (C-28), 70.5 (C-26), 73.7 (C-25), 78.2 (C-24), 78.6 (C-23), 80.7 (C-27), 96.5 (C-1' and C-7'), 101.3 (C-1" and C-7"), 109.9 (2CAr), 111.3 (2CAr), 121.3 (CH triazole), 122.0 (2CAr), 122.4 (2CAr), 123.3 (2CAr), 124.9 (C-3' and C-5'), 126.6 (2CAr), 128.2 (2CAr), 128.6 (2CAr), 132.9 (C-3" and C-5"), 140.1 (C-2' and C-6'), 140.7 (2CqAr), 142.2 (2CqAr), 142.7 (4CqAr), 143.8 (C-2" and C-6"), 146.6 (Ctriazole), 156.1 (C-4"), 163.1 (2C-1), 168.8 (C-4'), 170.3 (C=O), 171.9 (2C-11). HRMS (ESI): [$M^{2+}$] calcd for [C89H117N9O6$S^{2+}$] 719.9419, found 719.9417.

**N-3-S-PEG-c(RGDfK) heterodimer**

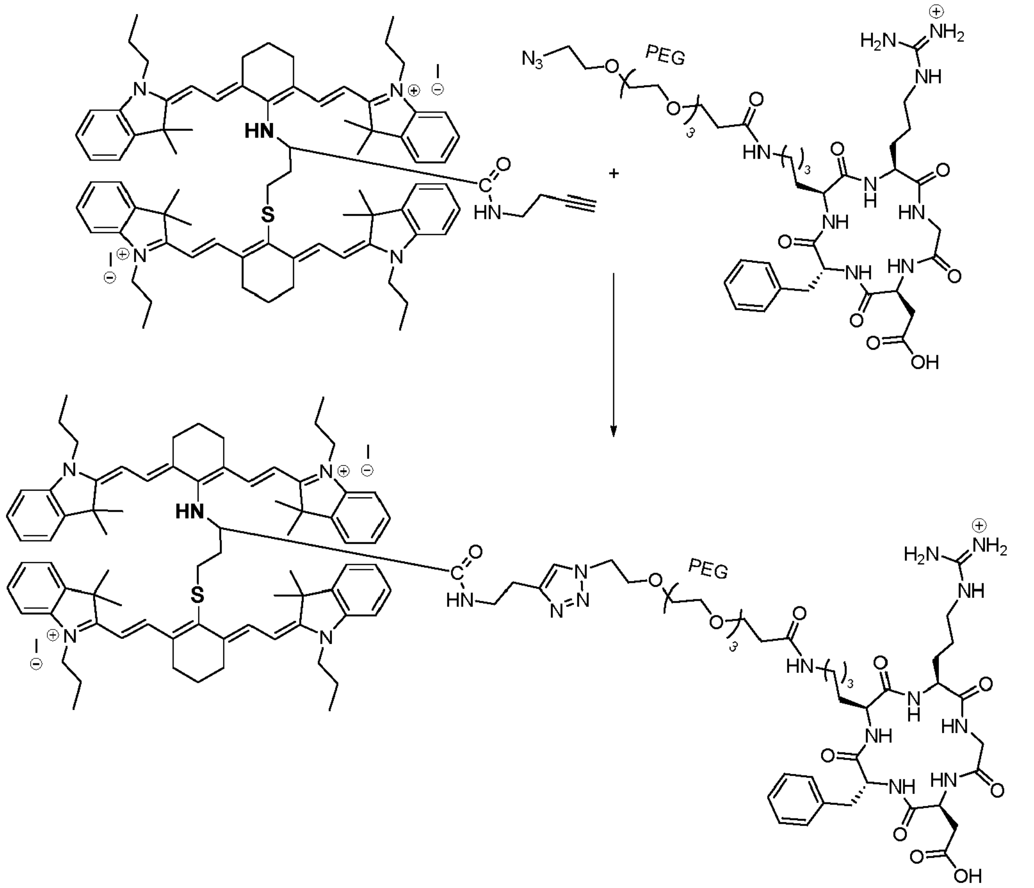

To a solution of N-3-S-alkyne (15.9 mg, 11 μmol, 1.0 eq.) and 10.9 mg of c(RGDfK)-PEG(4)-$N_3$ (11 μmol, 1.0 eq.) diluted in 0.5 mL of tetrahydrofurane, were added 37 μL of an aqueous solution of $CuSO_4.5H_2O$ (0.3M, 22 μmol, 1.0 eq.), 22 μL of an aqueous solution of sodium ascorbate (1M, 22 μmol, 2.0 eq.) and 0.45 mL of water. The mixture was stirred at 30°C for 16 h. The solvent was removed under *vacuum* and the residue was diluted in dichloromethane (10 mL) and washed with water (10 mL). The organic layer was dried over $MgSO_4$, and the solvent was removed under vacuum. The crude product was purified by steric exclusion gel filtration (Sephadex LH20) (eluent: MeOH) to afford the compound as a green/blue gum (14 mg, 20%).

HRMS (ESI): [$M^{3+}$] calcd for [$C_{118}H_{161}N_{18}O_{13}S^{3+}$] 690.0731, found 690.0715.

**N-3-S-COOH heterodimer-sulfonate**

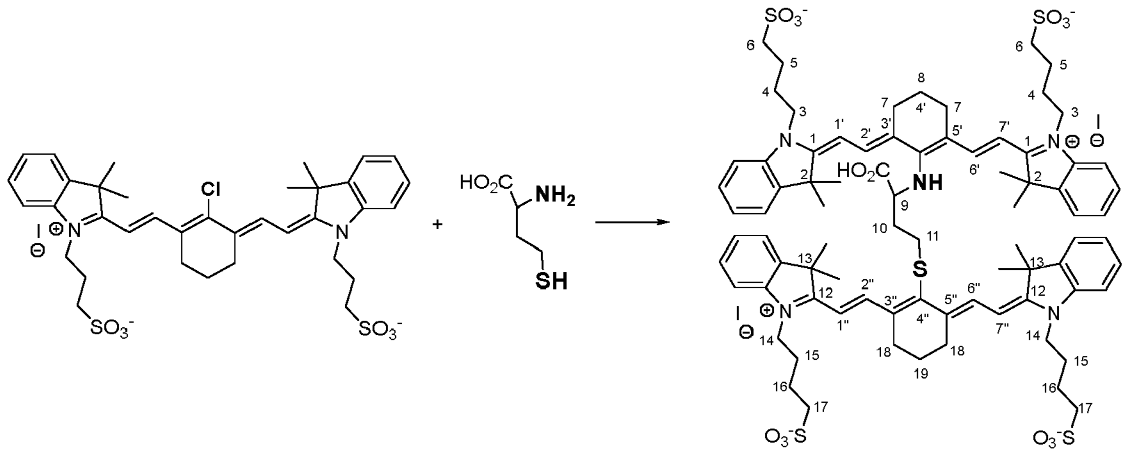

To a solution of 100 mg of commercial IR783 (0.13 mmol, 2 eq.) in freshly distilled dimethylformamide (1 mL) were added 28 μL of triethylamine (0.2 mmol, 3 eq.) and 9.0 mg of L-homocysteine (0.7 mmol, 1 eq.) at room temperature under inert atmosphere. The solution was stirred for 24 hours at room temperature then the solvent was removed under *vacuum.* The crude product was purified by flash chromatography on C18 column (25/75 to 50/50, ACN/$H_2O$) to give the product as a blue foam after lyophilization (100.6 mg, 49%).

$^1H$ NMR (400 MHz, $CD_3OD$): δ 1.60 (s, 6H, $CH_3$), 1.63 (s, 6H, $CH_3$), 1.69 (s, 6H, $CH_3$), 1.70 (s, 6H, $CH_3$), 1.76-1.87 (m, 20H, H-8, H-4, H-5, H-19, H-15, H-16), 2.22-2.30 (m, 1H, H-10a), 2.30-2.35 (m, 3H, 2H-7 or H-18 and H-10b), 2.60-2.77 (m, 8H, 6H-7 or H-18, and H-11), 2.78-2.82 (bt, 4H, J = 7.5 Hz, H-17 or H-6), 2.84 (bt, 4H, J = 7.5 Hz, H-17 or H-6), 3.89 (bt, 4H, *J* = 6.5 Hz, H-3 or H-14), 4.08 (bt, 4H, H-3 or H-14), 4.42-4.47 (m, 1H, H-9), 5.85 (d, 2H, *J* = 13.0 Hz, H-1', H-7'), 6.27 (d, 2H, *J* = 13.5 Hz, H-1", H-7"), 7.09-7.16 (m, 4H, HAr), 7.26 (t, 2H, *J* = 8.0 Hz, HAr), 7.33 (d, 6H, *J* = 8.0 Hz, HAr), 7.41 (d, 2H, HAr), 7.45 (d, 2H, HAr), 7.88 (d, 2H, H-2', H-6'), 8.85 (d, 2H, H-2", H-6"). $^{13}C$ NMR (100.6 MHz, $CD_3OD$): δ 22.3 (C-19), 23.1 (C-8), 23.6 (2C-5), 23.6 (2C-16), 26.1 (2C-7 or 2C-18), 26.7 (C-4 or C-15), 27.3 (C-4 or C-15), 27.3 (2C-7 or 2C-18), 28.6 ($4CH_3$), 28.6 ($2CH_3$), 28.7 ($2CH_3$), 29.7 (C-10), 34.5 (C-11), 44.0 (2C-3 or 2C-14), 44.9 (2C-3 or 2C-14), 47.9 (2C-2), 50.5 (2C-13), 51.8 (C-17 or C-6), 52.0 (C-17 or C-6), 62.3 (C-9), 97.1 (C-1' and C-7'), 102.4 (C-1" and C-7"), 110.7 (2CAr), 112.3 (2CAr), 122.4 (C-3' and C-5'), 123.1 (2CAr), 123.4 (2CAr), 124.6 (2CAr), 126.4 (2CAr), 129.6 (2CAr), 130.0 (2CAr), 135.3 (C-3" and C-5"), 140.5 (C-2' and C-6'), 141.7 (2CqAr), 142.3 (2CqAr), 143.6 (2CqAr), 144.2 (2CqAr), 147.3 (C-2" and C-6"), 157.3 (C-4"), 166.7 (2C-1), 170.2 (C-4'), 173.8 (C=O and 2C-12). HRMS (ESI): [$M^{2+}$] calcd for [$C_{80}H_{103}N_5O_{14}S_5^{2+}$] 758.8047, found 758.8032.

**N,O heterodimer**

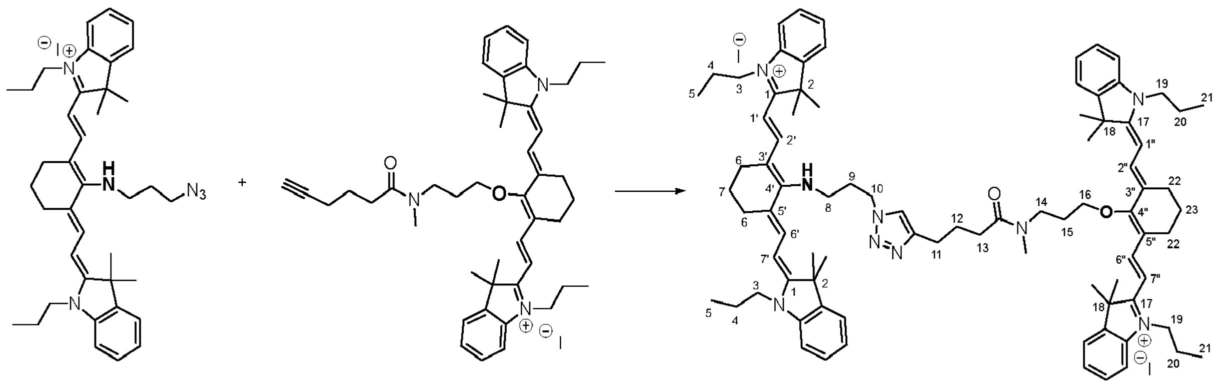

To a solution of N-cyanine derivative (62 mg, 0.085 mmol,1.3 eq.) and O-cyanine derivative (53 mg, 0.065 mmol, 1 eq.) in DMF (1 mL) were added 1 ml of water, copper (II) sulphate (0.026 mmol, 0.4 eq.), and sodium ascorbate (0.052 mmol, 0.8 eq.). The mixture was stirred at 40°C for 48 h in a sealed tube. The solvent was removed under *vacuum* and the residue was diluted in dichloromethane (10 mL) and washed with water (10 mL). The organic layer was dried over $MgSO_4$, and the solvent was removed under vacuum. The crude product was purified by flash chromatography on silica gel (eluent: AcOEt/DCM/MeOH 47/48/5) to afford the compound as a blue gum (32 mg, 31%).

$^1H$ NMR (400 MHz, $CDCl_3$) compound exists as a mixture of two rotamers, major rotamer is designated by * and minor by $^{\delta}$: δ 1.01 (t, 6H, $J$ = 7.5 Hz, H-5 or H-21), 1.05 (t, 6H, $J$ = 7.5 Hz, H-5 or H-21), 1.69 (s, 24H, $CH_3$), 1.74-1.93 (m, 12H, H-7, H-23, H-4, H-20), 2.01 (app qt, 2H, $J_{11,12}$ = $J_{13,12}$ = 7.5 Hz, H-12), 2.13-2.22 (m, 2H, H-15), 2.39 (t, 2H, H-13), 2.47 (bt, 4H, $J_{6,7}$ = 6.0 Hz, H-6), 2.58 (bt, 4H, $J_{22,23}$ = 6.0 Hz, H-22), 2.65-2.72 (app qt, 2H, $J_{9,8}$ = $J_{9,10}$ = 6.5 Hz, H-9), 2.78 (t, 2H, H-11), 3.00 (s, 3H, N-$CH_3^{\delta}$), 3.07 (s, 3H, N-$CH_3$*), 3.58-3.68 (m, 2H, H-14), 3.70-3.79 (m, 4H, H-3), 3.81-3.90 (m, 2H, H-8), 3.99-4.09 (m, 6H, H-19, H-16), 4.54 (t, 2H, H-10), 5.55 (d, 2H, $J$ = 13.0 Hz, H-1', H-7'), 6.02 (d, 2H, $J$ = 13.5 Hz, H-1'''*, H-7'''*), 6.07 (d, 2H, $J$ = 13.5 Hz, H-1''$^{\delta}$, H-7''$^{\delta}$), 6.81 (d, 2H, $J$ = 7.5 Hz, HAr), 7.01 (t, 2H, $J$ = 7.5 Hz, HAr), 7.09 (d, 2H, $J$ = 7.5 Hz, HAr), 7.18-7.26 (m, 6H, HAr), 7.34-7.39 (m, 4H, HAr), 7.73 (d, 2H, H-2', H-6'), 8.00 (d, 2H, H-2''$^{\delta}$, H-6''$^{\delta}$), 8.02 (s, 1H, H-triazole), 8.04 (d, 2H, H-2'''*, H-6'''*), 10.14 (bs, 1H, NH). $^{13}C$ NMR (100.6 MHz, $CDCl_3$): δ 11.8 (2C-5 or 2C-21), 11.9 (2C-5 or 2C-21), 20.1 (2C-4,), 20.9 (2C-20), 21.5 (C-7), 22.8 (C-23), 24.7 (2C-22), 24.8 (C-12), 25.0 (C-11), 25.9 (2C-6), 28.5 ($4CH_3$), 29.1 ($4CH_3$), 29.8 (C-15), 32.1 (C-9), 32.8 (C-13⍰ , 33.7 (N-$CH_3$⍰ ), 36.0 (N-$CH_3$*), 44.7 (2C-3), 45.2 (C-14), 46.0 (2C-19), 47.0 (C-8), 47.7 (2C-2), 48.1 (C-10), 49.1 (2C-18), 75.9 (C-16), 93.9, (C-1', C-7'), 99.6 (C-1'', C-7''), 108.3 (2CAr), 110.6 (2CAr), 120.3 (C-3', C-5'), 122.2 (2CAr), 122.4 (2CAr), 122.5 (CH-triazole), 123.5 (C-3'', C-5''), 125.1 (2CAr), 128.0 (4CAr), 128.8 (2CAr), 137.5 (C-2', C-6'), 140.8 (C-2'', C-6''), 141.1 (4CqAr), 142.5 (4CqAr), 144.7 (C-triazole), 166.9 (C-4'), 171.1 (2C-17), 171.4 (C-4''), 171.6 (2C-1, C=O). HRMS (ESI): [$M^{2+}$] calcd for [$C_{85}H_{111}N_9O_2{}^{z+}$] 644.9425, found 644.9455. IR ($\overline{\upsilon}$ (cm-1)): 3408, 2962, 2927, 2872, 1637, 1554, 1521, 1452, 1367, 1259, 1228, 1163, 1002, 927.

**O,O Homodimer**

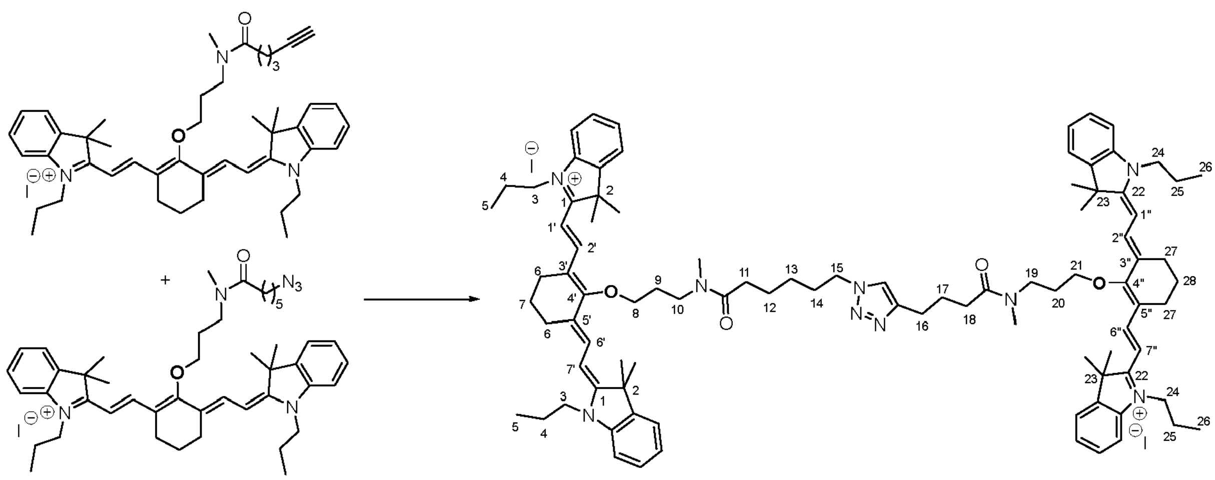

To a solution of O-cyanine-alkyne derivative (29 mg, 0.034 mmol, 1.2 eq.) and O-cyanine-azide derivative (23 mg, 0.028 mmol, 1 eq.) in DMF (1 mL) were added 135 μL of an aqueous solution of $CuSO_4.5H_2O$ (0.1 M, 0.0135 mmol, 0.4 eq.) and 271 μL of an aqueous solution of sodium ascorbate (0.1 M, 0.0271 mmol, 0.08 eq.). The mixture was stirred at 40°C for 1 h in a sealed tube under microwave irradiation. The solvent was removed under *vacuum* and the residue was diluted in dichloromethane (10 mL) and washed with water (10 mL). The organic layer was dried over $MgSO_4$, and the solvent was removed under vacuum. The crude product was purified by flash chromatography on silica gel (eluent: DCM/AcOEt 50/50 to DCM/MeOH 95/5) to afford the compound as a green gum (16 mg, 34%).

$^1H$ NMR (400 MHz, $CD_3CN$) compound exists as a mixture of two rotamers, major rotamer is designated by * and minor by $^{\delta}$: δ 1.00 (t, 12H, $J$ = 7.5 Hz, H-5 and H-26), 1.23-1.31 (m, 2H, H-13), 1.48-1.64 (m, 2H, H-12), 1.66 (s, 24H, $CH_3$), 1.75-1.89 (m, 16H, H-17, H-14, H-7, H-28, H-4, H-25), 2.13-2.30 (m, 4H, H-9, H-20), 2.25-2.45 (m, 4H, H-11, H-18), 2.58 (bt, 8H, $J$ = 6.0 Hz, H-6, H-27), 2.64-2.70 (m, 2H, H-16), 2.91-2.95 (m, 3H, N-$CH_3$), 3.00 (s, 3H, N-$CH_3$), 3.52-3.65 (m, 4H, H-19, H-10), 3.99-4.08 (m, 12H, H-24, H-3*$^{,\delta}$, H-8, H-21), 4.18-4.27 (m, 2H, H-15), 6.07 (d, 4H, $J$ = 13.5 Hz, H-1'*, H-7'*, H-1'''*, H-7'''*), 6.08 (d, 4H, $J$ = 13.5 Hz, H-1'$^{\delta}$ H-7'$^{\delta}$, H-1''$^{\delta}$, H-7''$^{\delta}$), 7.22 (t, 4H, $J$ = 7.5 Hz, HAr), 7.23 (d, 4H, $J$ = 7.5 Hz, HAr), 7.38 (t, 4H, $J$ = 7.5 Hz, HAr), 7.44-7.52 (m, 4H, HAr), 7.52 (s, 1H, H-triazole), 8.05 (d, 4H, $J$ = 13.5 Hz, H-2'$^{\delta}$, H-6'$^{\delta}$, H-2''$^{\delta}$, H-6''$^{\delta}$), 8.07 (d, 4H, $J$ = 13.5 Hz, H-2'*, H-6'* H-2'''*, H-6'''*). $^{13}C$ NMR (100.6 MHz, $CD_3CN$): δ 11.6 (2C-5, 2C-26), 21.4 (2C-4, 2C-25), 22.0 (C-7, C-28), 25.1 (C-12), 25.2 (2C-6, 2C-27), 25.7 (C-16), 25.8 (C-17 or C-14), 26.9 (C-13), 28.6 ($4CH_3$), 28.6 ($4CH_3$), 29.8 (C-9, C-20), 30.8 (C-17 or C-14), 33.2 (C-11 or C-18), 33.6 (N-$CH_3$), 33.6 (C-11 or C-18), 35.9 (N-$CH_3$), 45.3 (C-10 or C-19), 46.3 (2C-3, 2C-24), 47.5 (C-10 or C-19), 49.9 (2C-2, 2C-23), 50.5 (C-15), 75.9 (C-8 or C-21), 76.9 (C-8 or C-21), 100.3 (C-1'*, C-7'*, C-1'''*, C-7'''*), 100.4 (C-1'$^{\delta}$, C-7'$^{\delta}$, C-1''$^{\delta}$, C-7''$^{\delta}$), 111.8 (4CAr*), 111.9 (4CAr$^{\delta}$), 122.4 (CH-triazole), 123.3 (4CAr*), 123.7 (C-3'*, C-5'*, C-3'''*, C-5'''*), 123.8 (C-3'$^{\delta}$, C-5'$^{\delta}$, C-3''$^{\delta}$, C-5''$^{\delta}$), 125.6 (4CAr*), 125.7 (4CAr$^{\delta}$),

129.4 (4CAr), 141.5 (C-2'$^{\delta}$, C-6'$^{\delta}$, C-2"$^{\delta}$, C-6"$^{\delta}$), 141.7 (C-2'*, C-6'*, C-2"*, C-6"*), 142.0 (4CqAr), 143.7 (4CqAr, C-triazole), 171.8 (C-4', C-4"), 172.8 (2C-1, 2C-22, 2C=O). HRMS (ESI): [$M^{2+}$] calcd for [$C_{92}H_{123}N^{9}O_4{}^{2+}$] 708.9844, found 708.9868. IR ($\bar{\upsilon}$ (cm-1)): 3410, 2926, 2872, 2096, 1708, 1631, 1552, 1508, 1450, 1394, 1352, 1311, 1165, 1001, 927, 840, 794.

**N-8-N homodimer**

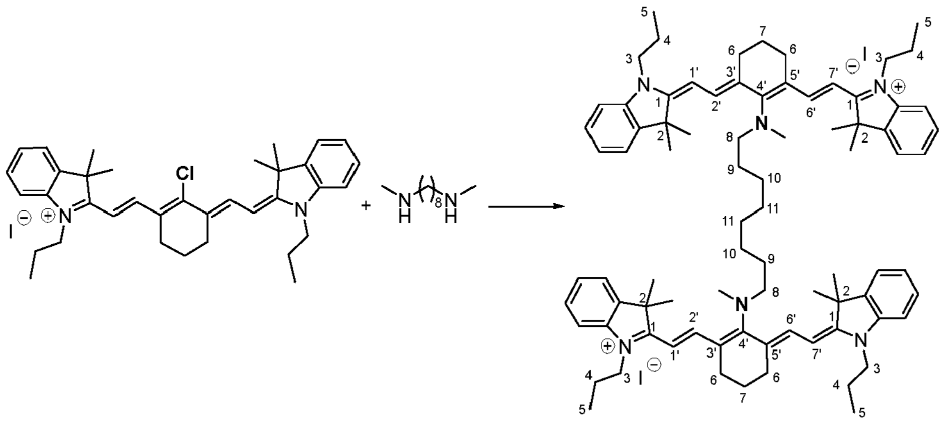

To a solution of 88 mg of commercial IR780 (0.13 mmol, 2 eq.) in freshly distilled acetonitrile (1 mL) were added 18 mg of potassium carbonate (0.13 mmol, 2 eq.) and 11.3 mg of N,N'-dimethyl-1,8-octanediamine (0.0657 mmol, 1 eq.) at room temperature under inert atmosphere. The solution was stirred for 48 hours at reflux in a sealed tube. The solvent was removed under *vacuum,* the residue was diluted in dichloromethane (10 mL) and washed with water (10 mL). The organic layer was dried over $MgSO_4$, and the solvent was removed under vacuum. The crude product was triturated in a dichloromethane/pentane 1/1 mixture and was filtrated to give the product as a blue solid (24 mg, 25%).

Mp : 142-144°C ; $^{1}H$ NMR (400 MHz, CDCl3): $\delta$ 1.04 (t, 12H, $J_{4,5}$ = 7.5 Hz, H-5), 1.29-1.39 (m, 8H, H-10, H-11), 1.63 (s, 24H, $CH_3$), 1.76-1.88 (m, 16H, H-7, H-4, H-9), 2.47 (t, 8H, $J_{6,7}$ = 6.0 Hz, H-6), 3.47 (s, 6H, N-$CH_3$), 3.72-3.80 (m, 4H, H-8), 3.86 (t, 8H, $J_{3,4}$ = 6.5 Hz, H-3), 5.76 (d, 4H, $J$ = 13.5 Hz, H-1', H-7'), 6.93 (d, 4H, $J$ = 7.5 Hz, HAr), 7.10 (t, 4H, $J$ = 7.5 Hz, HAr), 7.27-7.34 (m, 8H, HAr), 7.49 (d, 4H, H-2', H-6'). $^{13}C$ NMR (100.6 MHz, $CDCl_3$): $\delta$ 11.9 (4C-5), 20.5 (4C-4), 22.0 (2C-7), 25.0 (4C-6), 27.0 (2C-10 or 2C-11), 29.6 (2C-10 or 2C-11 or 2C-9), 29.6 (8$CH_3$), 29.7 (2C-10 or 2C-11 or 2C-9), 45.3 (4C-3), 45.8 (2N-$CH_3$), 48.2 (4C-2), 59.6 (2C-8), 95.7 (2C-1' and 2C-7'), 109.3 (4CAr), 122.4 (4CAr), 123.5 (4CAr), 123.8 (2C-3' and 2C-5'), 128.5 (4CAr), 140.3 (4CqAr), 142.0 (2C-2' and 2C-6'), 143.1 (4CqAr), 168.8 (4C-1), 175.2 (2C-4'). HRMS (ESI): [$M^{2+}$] calcd for [$C_{82}H_{110}N_6{}^{2+}$] 589.4391, found 589.4375. IR ($\bar{\upsilon}$ (cm-1)): 2964, 2927, 1550, 1512, 1452, 1373, 1348, 1274, 1165, 1099, 933.

**N-3-N homodimer**

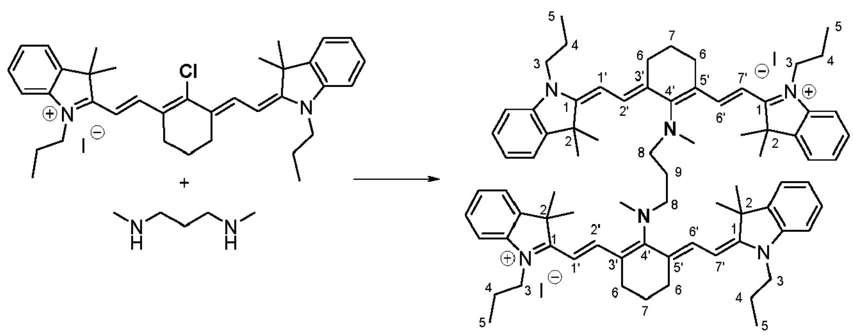

To a solution of 52 mg of commercial IR780 (0.078 mmol, 2 eq.) in freshly distilled acetonitrile (1 mL) were added 11 mg of potassium carbonate (0.078 mmol, 2 eq.) and 4.0 mg of N,N'-dimethyl-1,3-propanediamine (0.039 mmol, 1 eq.) at room temperature under inert atmosphere. The solution was stirred for 48 hours at reflux. The solvent was removed under *vacuum* and the crude product was purified by flash chromatography on silica gel (eluent: DCM/MeOH 93/7) to afford the

compound as a blue gum (11 mg, 21%).

$^1H$ NMR (400 MHz, $CDCl_3$): δ 1.01 (t, 12H, $J_{4,5}$ = 7.5 Hz, H-5), 1.65 (s, 24H, $CH_3$), 1.76-1.87 (m, 14H, H-7, H-4, H-9), 2.47 (t, 8H, $J_{6,7}$ = 6.0 Hz, H-6), 3.65 (s, 6H, N-$CH_3$), 3.86 (t, 8H, $J_{3,4}$ = 6.5 Hz, H-3), 3.88-3.95 (m, 4H, H-8), 5.77 (d, 4H, *J* = 13.0 Hz, H-1', H-7'), 6.91 (d, 4H, *J* = 8.0 Hz, HAr), 7.09 (t, 4H, *J* = 8.0 Hz, HAr), 7.25-7.31 (m, 8H, HAr), 7.53 (d, 4H, H-2', H-6'). $^{13}C$ NMR (100.6 MHz, $CDCl_3$): δ 11.9 (4C-5), 20.5 (4C-4 and C-9), 21.9 (2C-7), 25.3 (4C-6), 29.8 (8$CH_3$), 45.4 (4C-3), 46.6 (2N-$CH_3$), 48.2 (4C-2), 56.5 (2C-8), 96.1 (2C-1' and 2C-7'), 109.3 (4CAr), 122.3 (4CAr), 123.5 (4CAr), 124.3 (2C-3' and 2C-5'), 128.4 (4CAr), 140.4 (4CqAr), 141.8 (2C-2' and 2C-6'), 143.1 (4CqAr), 168.8 (4C-1), 174.8 (2C-4'). HRMS (ESI): [$M^{2+}$] calcd for [$C_{77}H_{100}N_6^{2+}$] 554.3999, found 554.4018. IR ($\bar{\upsilon}$ (cm-1)): 2989, 2970, 1550, 1508, 1452, 1373, 1348, 1197, 1165, 1124, 1097, 1002, 931.

## Fluorescence quantum yield measurement protocol

To determine the molar absorption coefficient (ε, $M^{-1}cm^{-1}$), absorption spectra were recorded in diluted solution (μM) in DMSO (or in $H_2O$/DMSO 95/5) at 298 K and linear relationship with concentration according to Lambert-Beer law was verified with $R^2$ values between 0.995 - 0.999. All absorbance spectra were recorded on a Lambda 1050 UV/VIS/NIR spectrophotometer (Perkin Elmer) in quartz cuvette with 1 cm optical path length using a Peltier™ thermostatted sample holder.

The relative fluorescence quantum yields $\Phi_{fuo}$ were measured at 298 K in diluted solutions with an absorbance lower than 0.1 using the following equation

$$\phi_{fluo,x} = \phi_{fluo,ref}\left(\frac{Grad_x}{Grad_{ref}}\right)\left(\frac{n_x^2}{n_{ref}^2}\right)$$

where $\Phi_{fluo}$ is the fluorescence quantum yield, Grad is the gradient from the plot of integrated fluorescence intensity vs absorbance, n the refractive index of the solvent ($n_{water}$ = 1.3291, $n_{DMSO}$ = 1.477, $n_{EtOH}$ = 1.3579), and the subscripts x and ref denote sample and reference. First, the fluorescence quantum yield of the commercial IR 780 in DMSO (or in $H_2O$/DMSO 95/5) was measured relative to IR 780 in EtOH for which $\Phi_{fluo,ref}$ = 0.08. Then, the fluorescence quantum yield of the studied compound in DMSO (or in $H_2O$/DMSO 95/5) were determined. Excitation of reference and sample compounds was performed at the same wavelength ($\lambda_{ex}$ = 750 nm). All fluorescence spectra were recorded on a Fluorolog 3 spectrofluorometer (Horiba, France) in a quartz cuvette with 1 cm optical path length using a Peltier™ thermostatted sample holder.

## Protocol for evaluation of Photoacoustic properties

Photoacoustic imaging was performed on a PAFT Tritom tomograph (PhotoSound, Houston, USA) using a fibered nanosecond pulsed laser (20 Hz repetition rate, 3-5 ns pulse duration, 160 mJ max OPO output energy, PhotoSonus, Ekspla, Lithuania) tunable between 330-1320 nm and a curved multichannel transductor. The samples solubilized in DMSO or water were loaded in PTFE tube (inner diameter 0.81 mm, Photosound, Houston, USA) and mounted on a rotating holder for immersion into deionized water at 36 °C. The standard used was aqueous $CuSO_4$ which absorbs between 650-1000 nm, non-fluorescent, non-scattering and does not undergo photobleaching or photodegradation under irradiation in this optical region. Raw data treatment was performed with Matlab software (version R2022a) using a code provided by the PA system manufacturer, and processed with 3Dslicer software (version 5.0.2) to measure the averaged ROI values as drawn areas on each samples' tube. The ROI values were normalized to background deionized water.

## Protocol for evaluation of Photothermal conversion efficiency

The home-made irradiation and photothermal measurement setup are composed of a highly stable continuous-wave 808 nm infrared diode laser (MDL-H-808, CNILasers - China) with tunable output power between 0 and 3 W. It was coupled to a 400μm@1m optical fiber positioned at 79 mm perpendicularly relative to the sample irradiated surface and hitting the center of the well (diameter of 0.68 cm). The laser power at the level of sample was measured by a power meter with thermal power sensor (PM16-405, Thorlabs, France) and the corresponding irradiance (power density) was calculated using the irradiated sample surface (0.36 $cm^2$). The temperature variation was followed by a thermal camera (optical resolution of 382 × 288 pixels, 80 Hz frame rate, Xi400, Optris, France) positioned above the sample at 89 mm with a ~16° angle between the objective axis and the laser beam axis. The thermal camera measured directly the temperature on the sample surface for which the emissivity of liquid (water) of 0.95 was applied. A 96-well plate (plastic) as sample holder was used containing 200 μL of solution of each compound at different concentrations (4-40 μM) in DMSO (or in

$H_2O$/DMSO 95/5), the height of the solution corresponds to a light path of 6 mm. The whole system was isolated from external light and heat by a black heavy duty foam board cage (5 mm thick). Measurements were conducted in an air-conditioned room during all experiments. Using this setup, solutions were irradiated for 600 s followed, after extinction of the laser, by 600 s of cooling period. The temperature of the solution was recorded with an accuracy of 0.1 °C every 0.5 s. The resistance to photothermal fatigue was studied by performing 4 cycles (irradiation/cooling to room temperature) as described above. The light-to-heat (photothermal) conversion efficiency ($\eta$) was calculated according to the method reported by Wang and compared to a standard for which $\eta$ can be assumed to be 100%. The non-fluorescent and non-scattering reference solutions were $CuSO_4$ in water or Nigrosin in DMSO. The experimental setup described above was optimized with these two references solutions such that $\Delta T$ measured at different concentrations and laser power densities (0.3-1.0 $Wcm^{-2}$) gave the highest photothermal conversion efficiency (95-100%). All measurements were repeated at least three times on fresh sample, and the reference solution was measured after each compound solution.

**Results**

The results are provided in the following table

| | Photophysical properties | | | | | Photoacoustic signal[b] | | Photothermal properties[c] $\Delta T_{max}$ (°C) |
|---|---|---|---|---|---|---|---|---|
| | $\lambda_{abs}$ (nm) | $\lambda_{em}{}^{a}$ (nm) | $\varepsilon$ ($M^{-1}$ $cm^{-1}$) | $\Phi_{fluo}$ | B ($M^{-1}$ $cm^{-1}$) | at $\lambda_{ex}$ 700 nm | at $\lambda_{ex}$ 808 nm | |
| N-2-S | 798 | 817 | 189300 | <1 | <1900 | >ICG strong | >ICG strong | 95[c] |
| N-6-S | 801 | 814 | 144000 | 2 | 2880 | >ICG strong | >ICG strong | 81[c] |
| N-3-S-PEG | 800 | 812 | 150100 | nd | nd | nd | nd | 15[d] |
| N-3-S-glucose | 800 | 814 | 91000 | nd | nd | nd | nd | 15[d] |
| N,O | 774 | 799 | 217600 | 8 | 17400 | nd | nd | 42[c] |
| O,O | 772 | 790 | 374300 | 17 | 63600 | nd | nd | 68[c] |
| N-8-N | 680 | 797 | 102000 | 4 | 4100 | >ICG strong | < ICG low | 32[c] |
| N-3-N | 688 | 803 | 118000 | 3 | 3500 | >ICG strong | < ICG low | 21[c] |

nd: not determined
[a] $\lambda_{ex}$= 750 nm, DMSO
[b] [ICG] = 40 $\mu$M, [dimer] = 20 $\mu$M, DMSO, T = 36°C
[c] 20 $\mu$M, 808 nm laser, 1 $Wcm^{-2}$, 5 min, DMSO
[d] 4 $\mu$M, 808 nm laser, 0.5 $Wcm^{-2}$, 5 min, DMSO

**Claims**

**1.** Compounds of formula (I)

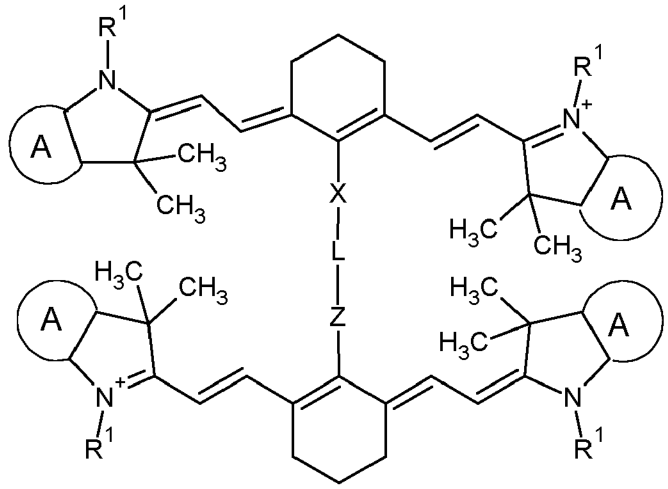

, 2 $D^-$ (I)

wherein

- A represents a phenyl or naphtyl group, optionally substituted;
- D, each identical or different, represents an anion;
- $R^1$ represents a propyl group or a butyl-$SO_3M$ group with M is H, an ammonium group or a cation;
- X is NH, N(Alk), S or O, with Alk is an alkyl group, linear or branched, comprising from 1 to 10 carbon atoms, preferably from 1 to 5 carbon atoms, preferably $CH_3$,
- Z is NH, N(Alk), S or O, with Alk is an alkyl group, linear or branched, comprising from 1 to 10 carbon atoms, preferably from 1 to 5 carbon atoms, preferably $CH_3$,
- L is $(CH_2)_n$-Ph-$(CH_2)_{n'}$ wherein the phenyl is substituted by a COR' group; $(CH_2)_m$-(triazole)-$(CH_2)_P$-C(O) N($CH_3$)$(CH_2)_{m'}$; alkyl linear or branched comprising from 1 to 8 carbon atoms; alkyl, linear or branched, comprising from 1 to 8 carbon atoms and substituted by one COR' group; $(CH_2)_m$-N($CH_3$)-C(O)-$(CH_2)_q$-(triazole)-$(CH_2)_p$-C(O)-N($CH_3$)-$(CH_2)_{m'}$,

  - p is an integer between 1 and 5, preferably between 2 and 4,
  - q is an integer between 1 and 5, preferably between 2 and 4,
  - m is an integer between 1 to 8,
  - m' is an integer between 1 to 8,
  - n is an integer between 0 and 2,
  - n' is an integer between 0 and 2;
  - R' is OH or R;
  - R is is NH-$(CH_2)_r$-triazole-$(CH_2)_2$-O-$(CH_2$-$CH_2$-O$)_x$-$CH_2CH_2$C(O)NH-W; NH$(CH_2)_r$-triazole-$(CH_2)_s$-(polyhydroxylated 5 or 6-cycloalkyl comprising an oxygen atom)-OW; NH$(CH_2)_r$-triazole-$(CH_2)_s$-(polyhydroxylated 5 or 6-cycloalkyl comprising an oxygen atom)-OH; NH-$(CH_2)_r$-triazole-$(CH_2)_2$-O-$(CH_2$-$CH_2$-O$)_x$ $(CH_2)_2$-COOH; NH-$(CH_2)_r$-triazole-$(CH_2)_2$-O-$(CH_2$-$CH_2$-O$)_x$$(CH_2)_2$-C(O)-W; NH-alkyl-C=CH wherein the alkyl is linear or branched and comprises from 2 to 5 carbon atoms; NH-alkyl-$N_3$ wherein the alkyl is linear or branched and comprises from 2 to 5 carbon atoms; NH-alkyl-NH-Q wherein the alkyl is linear or branched and comprises from 2 to 5 carbon atoms; NH-$(CH_2)_2$-O-$(CH_2$-$CH_2$-O$)_x$-$CH_2CH_2$-COOH; NH-$(CH_2)_2$-O-$(CH_2$-$CH_2$-O$)_x$-$CH_2CH_2$-NH-Q; NH-$(CH_2)_2$-O-$(CH_2$-$CH_2$-O$)_x$-$CH_2CH_2$-CO-NH-W; NH-$(CH_2)_2$-O-$(CH_2$-$CH_2$-O$)_X$-$CH_2CH_2$-NH-W; W; PEG group; solubilizing group;

  Q is H or a protective group;
  r is an integer between 2 to 5,
  s is an integer between 2 to 5,
  x is an integer between 2 to 8,
  W is a bioactive compound or a group comprising a bioactive compound;

with the proviso that when X=Z=O, NH or S, L is not an alkyl and its pharmaceutically acceptable salts.

**2.** Compound according to claim 1, wherein when X=Z L is not $CH_2$-Ph-$CH_2$.

**3.** Compound according to claim 1 or 2, wherein X is NH, S or O, Z is NH, S or O and X and Z are different.

**4.** Compound according to any one of claims 1 to 3, wherein A is a phenyl group.

**5.** Compound according to anyone of claims 1 to 4, chosen among the following compounds:

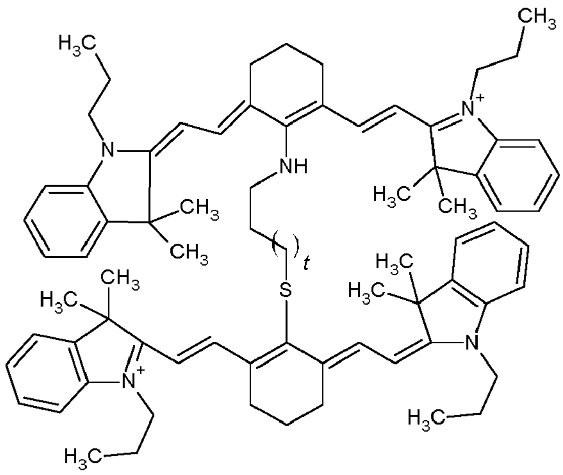

, $2D^-$

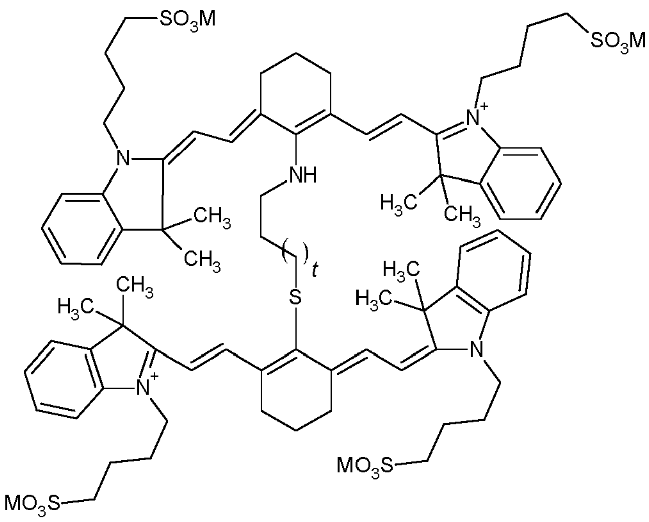

, $2D^-$

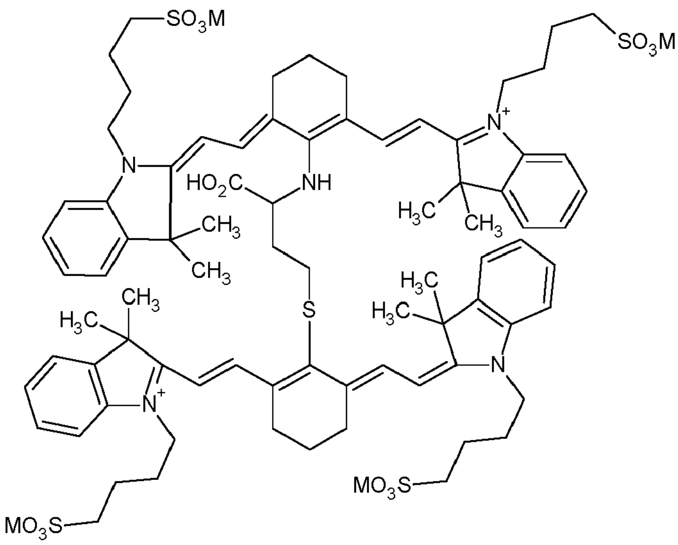

, $2D^-$

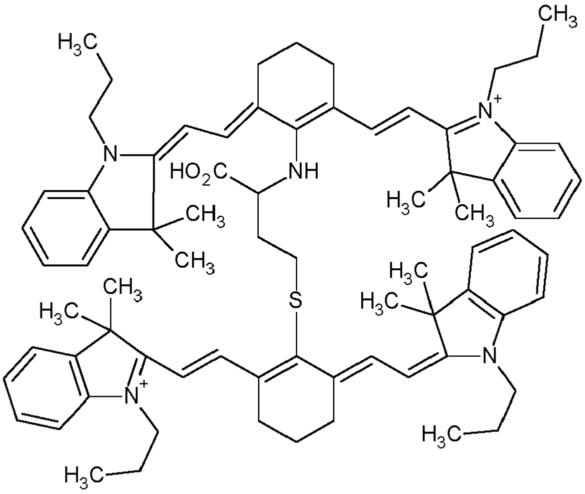

, $2D^-$

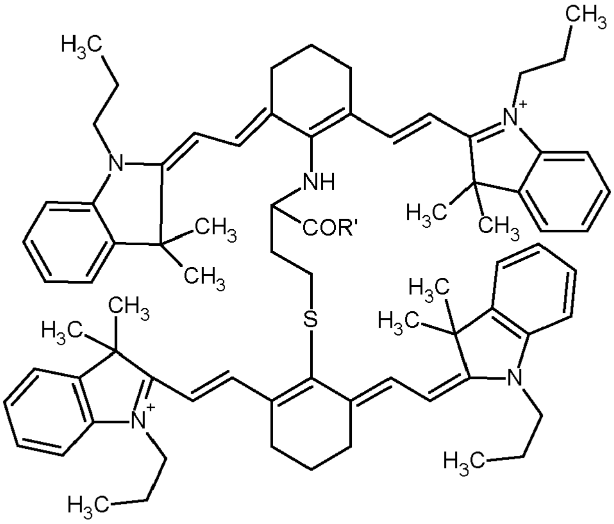

, $2D^-$

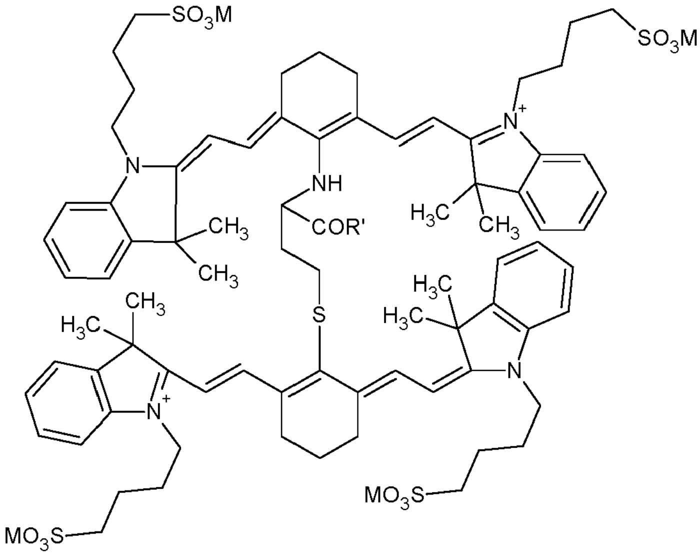

, $2D^-$

R' is $NH-(CH_2)_{1-3}-(CH_2)-C{\equiv}CH$, $NH-(CH_2)_{1-3}-(CH_2)$-NH-protection, $NH-(CH_2)_{1-3}-CH_2$-(triazole)-$CH_2-CH_2-O(CH_2-CH_2-O)_{3-7}-CH_2-CH_2-COOH$, $NH-(CH_2)_{1-3}-CH_2$-(triazole)-$CH_2-CH_2-CH_2$-(polyhydroxylated 5 or 6-cycloalkyl comprising an oxygen atom)-OH, $NH-(CH_2)_{1-3}-CH_2$-(triazole)-$CH_2-CH_2-CH_2$-(polyhy-

droxylated 5 or 6-cycloalkyl comprising an oxygen atom)-OW, NH-$(CH_2)_{1-3}$-$CH_2$-(triazole)-$CH_2$-$CH_2$-O($CH_2$-$CH_2$-O)$_{3-7}$-$CH_2$-$CH_2$-CONHW; NH-$(CH_2)_2$-O-($CH_2$-$CH_2$-O)$_x$-$CH_2CH_2$-COOH; NH-$(CH_2)_2$-O-($CH_2$-$CH_2$-O)$_x$-$CH_2CH_2$-NH-Q; NH-$(CH_2)_2$-O-($CH_2$-$CH_2$-O)$_x$-$CH_2CH_2$-CO-NH-W; NH-$(CH_2)_2$-O-($CH_2$-$CH_2$-O)$_x$-$CH_2CH_2$-NH-W; x, Wand Q being as defined in the preceding claims ;

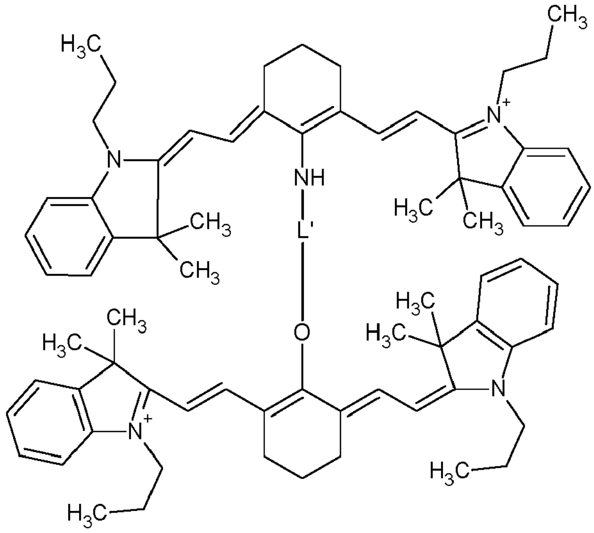

, $2D^-$

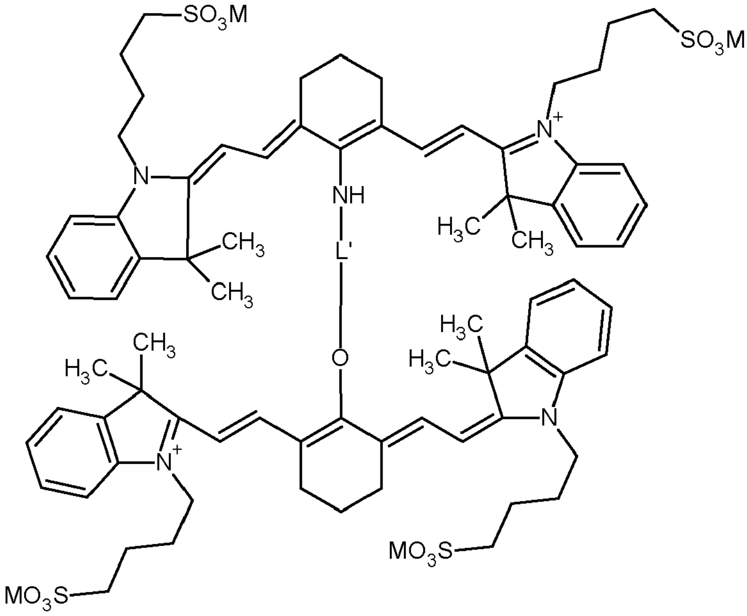

, $2D^-$

L' is $(CH_2)_m$-(triazole)-$(CH_2)_P$-C(O)N($CH_3$)$(CH_2)_{m'}$; p, m and m' being as defined in the preceding claims

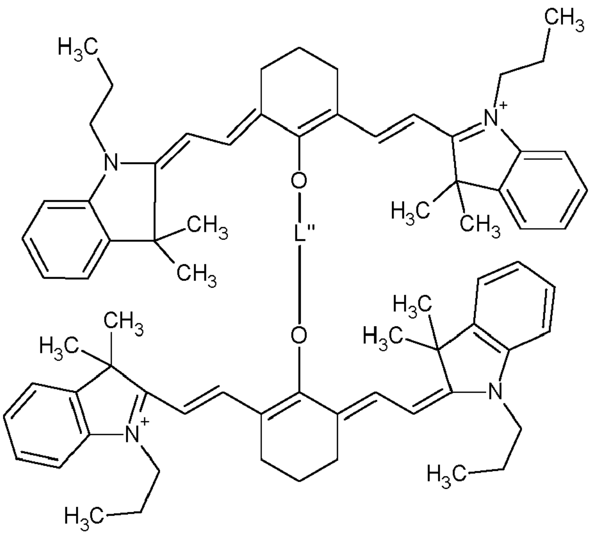

, $2D^-$

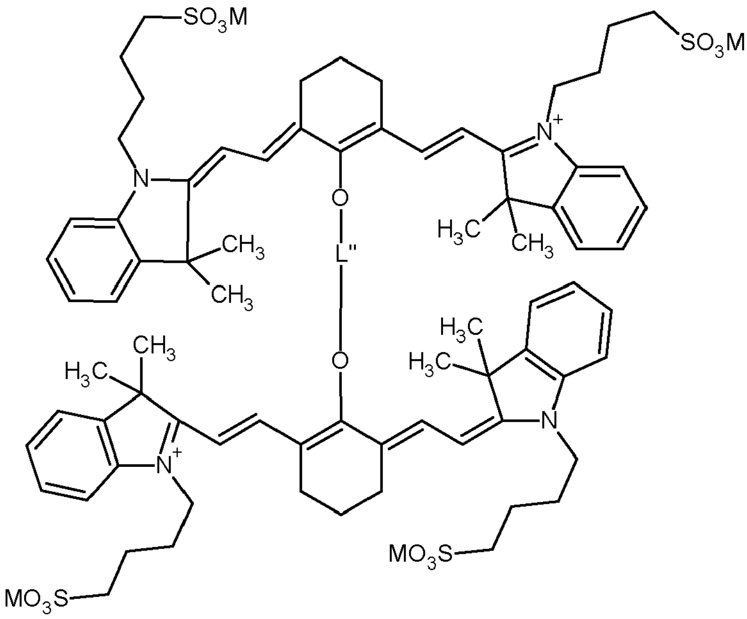

, $2D^-$

L" is $(CH_2)_m-N(CH_3)-C(O)-(CH_2)_q$-(triazole)-$(CH_2)_p-C(O)-N(CH_3)-(CH_2)_{m'}$, p, q, m and m' being as defined in the preceding claims

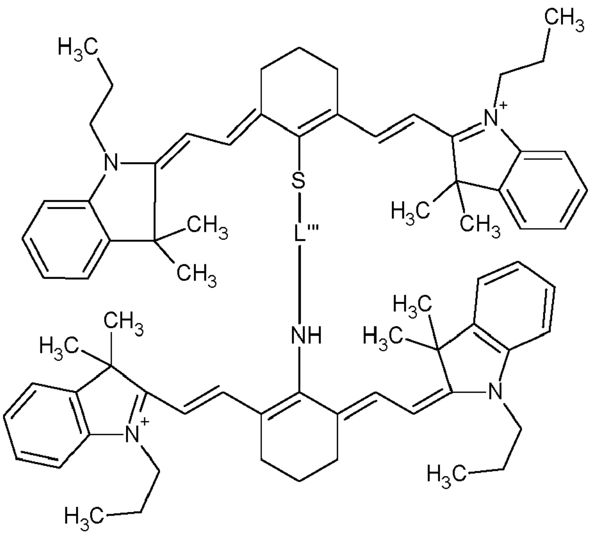

, $2D^-$

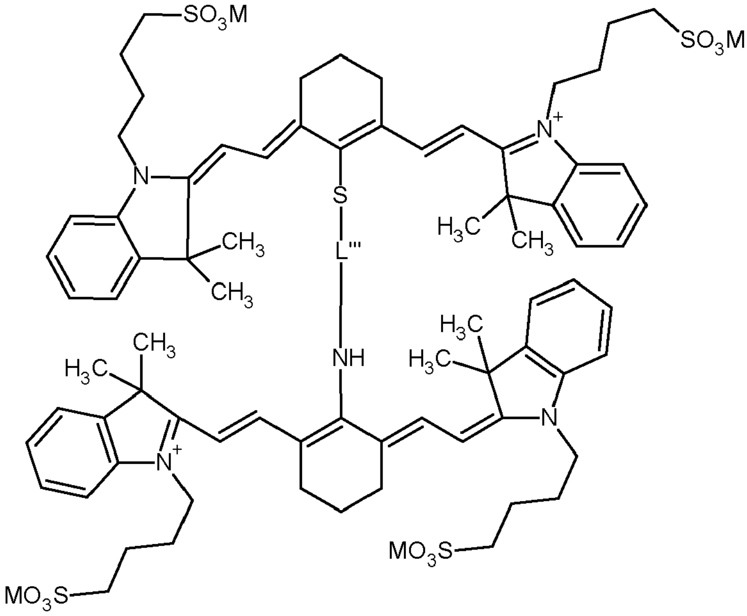

, $2D^-$

L‴ is $(CH_2)_n$-Ph-$(CH_2)_{n'}$ wherein the phenyl is substituted by a COR' group; R' is OH or R, preferably OH; R, n and n' being as defined in the preceding claims
wherein t is an integer from 0 to 6, D each identical or different represents an anion, preferably I; M is H, an ammonium group or a cation, preferably Na or K.

6. Composition comprising at least one compound of formula (I) and optionally at least one pharmaceutically acceptable excipient.

7. Compound according to any one of claims 1 to 4 or composition according to claim 5 for use for the diagnosis of tumor by fluorescent imaging and/or photoacoustic imaging.

8. Compound according to any one of claims 1 to 4 or composition according to claim 5 for use for the treatment of cancer or microbial infection.

9. Compound or composition according to claim 7 for use for the treatment of Glioblastoma, prostate cancer, breast cancer and melanoma.

10. Compound according to any one of claims 1 to 4 or composition according to claim 5 for use for phothermal therapy,

preferably for the treatment of cancer, preferably for the treatment of Glioblastoma, prostate cancer, breast cancer and melanoma.

**11.** Compound or composition according to claim 8 for use for photothermal therapy guides by fluorescent and photoacoustic imaging.

Europäisches Patentamt
European Patent Office
Office européen des brevets

# EUROPEAN SEARCH REPORT

Application Number
EP 24 30 5532

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YANXIAN HOU ET AL: "Synergistic Inter- and Intramolecular Aggregation of Dimeric Cyanine Dyes Affords Highly Efficient In Vivo Self-Delivery and Photothermal Therapy", ADVANCED FUNCTIONAL MATERIALS, WILEY - V C H VERLAG GMBH & CO. KGAA, DE, vol. 34, no. 32, 22 March 2024 (2024-03-22), page n/a, XP072683277, ISSN: 1616-301X, DOI: 10.1002/ADFM.202316452 * page 1 - page 2 * * page 10 * ----- | 1-11 | INV. C09B23/01 C09B23/08 A61P35/00 |
| A | KR 2018 0011423 A (UNIV EWHA IND COLLABORATION [KR]) 1 February 2018 (2018-02-01) * page 3; claim 1; compound 1 * ----- | 1-11 | |
| A | ZHAO XIUJIE ET AL: "A Tumor-Targeting Near-Infrared Heptamethine Cyanine Photosensitizer with Twisted Molecular Structure for Enhanced Imaging-Guided Cancer Phototherapy", JOURNAL OF THE AMERICAN CHEMICAL SOCIETY, vol. 143, no. 49, 3 December 2021 (2021-12-03), pages 20828-20836, XP093198638, ISSN: 0002-7863, DOI: 10.1021/jacs.1c09155 * page 20829; compound T780T * ----- | 1-11 | TECHNICAL FIELDS SEARCHED (IPC): C09B A61P |
| A | EP 1 129 861 A1 (FUJI PHOTO FILM CO LTD [JP]) 5 September 2001 (2001-09-05) * page 5; compounds IR-5, IR-6, IR-7, IR-8 * ----- -/-- | 1-11 | |

The present search report has been drawn up for all claims

| Place of search | Date of completion of the search | Examiner |
|---|---|---|
| Munich | 4 December 2024 | Jeanjean, Fabien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

5

Europäisches Patentamt
European Patent Office
Office européen des brevets

# EUROPEAN SEARCH REPORT

Application Number
EP 24 30 5532

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JP H08 95197 A (FUJI PHOTO FILM CO LTD) 12 April 1996 (1996-04-12) * page 7; compounds 3, 4 * ----- | 1-11 | |
| A | CN 110 283 474 A (UNIV BEIJING) 27 September 2019 (2019-09-27) * page 2; claim 1; compound III * ----- | 1-11 | |

TECHNICAL FIELDS SEARCHED (IPC)

The present search report has been drawn up for all claims

| Place of search | Date of completion of the search | Examiner |
|---|---|---|
| Munich | 4 December 2024 | Jeanjean, Fabien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 24 30 5532

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [X] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [ ] None of the further search fees have been paid within the fixed time limit. The present European search report has been drawn up for those parts of the European patent application which relate to the invention first mentioned in the claims, namely claims:

- [ ] The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 24 30 5532

The Search Division considers that the present European patent application does not comply with the requirements of unity of invention and relates to several inventions or groups of inventions, namely:

1. claims: 1-11(partially)

Compound of formula (I) wherein L is (CH2)n-Ph-(CH2)n and its related subject-matter

---

2. claims: 1-11(partially)

Compound of formula (I) wherein L is (CH2)m-(triazole)-(CH2)p-C(O)N(CH3)(CH2)m or (CH2)m-N(CH3)-C(O)-(CH2)q-(triazole)-(CH2)p-C(O)N(CH3)(CH2)m and its related subject-matter

---

3. claims: 1-11(partially)

Compound of formula (I) wherein L is optionally substituted alkyl and its related subject-matter

---

## ANNEX TO THE EUROPEAN SEARCH REPORT ON EUROPEAN PATENT APPLICATION NO. EP 24 30 5532

This annex lists the patent family members relating to the patent documents cited in the above-mentioned European search report. The members are as contained in the European Patent Office EDP file on
The European Patent Office is in no way liable for these particulars which are merely given for the purpose of information.

04-12-2024

| Patent document cited in search report | Publication date | Patent family member(s) | Publication date |
|---|---|---|---|
| KR 20180011423 A | 01-02-2018 | NONE | |
| EP 1129861 A1 | 05-09-2001 | AT E281310 T1 | 15-11-2004 |
| | | DE 60106796 T2 | 27-10-2005 |
| | | EP 1129861 A1 | 05-09-2001 |
| | | JP 4132547 B2 | 13-08-2008 |
| | | JP 2001242613 A | 07-09-2001 |
| | | US 2002028404 A1 | 07-03-2002 |
| JP H0895197 A | 12-04-1996 | NONE | |
| CN 110283474 A | 27-09-2019 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Remington's Pharmaceutical Sciences. Mack Publishing Company, 1985, 1418
- **P.H. STAHL** ; **C.G. WERMUTH**. Handbook of Pharmaceutical salts - Properties, Selection and Use. Wiley-VCH, 2002
- **S.M. BERGE et al.** Pharmaceutical Salts. *J. Pharm. Sci*, 1977, vol. 66, 1-19
- Remington's Pharmaceutical Sciences. Mack Publishing Company, 1985
- Merck Index. The pharmacological basis of therapeutics. Pergamon press, 1990